# EUROPEAN PATENT APPLICATION

(11) **EP 4 506 457 A1**
(43) Date of publication of application: **12.02.2025**
(21) Application number: 23775123.5
(22) Date of filing: 27.03.2023
(51) Int. Cl.: C12N 15/13

(54) **ANTI-CD39 ANTIBODY**

(30) Priority: 25.03.2022 JP 2022050874; 24.10.2022 JP 2022170178
(71) Applicant: Brightpath Biotherapeutics Co., Ltd., Kawasaki-shi, Kanagawa 210-0821 (JP)
(72) Inventor: NAKAMURA, Norihiro, Kawasaki-shi, Kanagawa 210-0821 (JP); MIE, Motoya, Kawasaki-shi, Kanagawa 210-0821 (JP); OBONAI, Toshifumi, Kawasaki-shi, Kanagawa 210-0821 (JP); BAN, Haruka, Yokohama-shi, Kanagawa 235-0023 (JP); MATSUMOTO, Noriko, Yokohama-shi, Kanagawa 231-0063 (JP); SHIRAISHI, Mamoru, Tochigi-shi, Tochigi 329-4405 (JP); MATSUMURA, Haruka, Kawasaki-shi, Kanagawa 210-0821 (JP)
(74) Representative: Gulde & Partner
(86) International application number: PCT/JP2023/012119
(87) International publication number: WO 2023/182530

(57) **Abstract**

The present invention addresses the problem of obtaining a superior anti-CD39 antibody. Provided is an antibody or antigen-binding fragment capable of binding to CD39, comprising heavy chain CDRs 1-3 and light chain CDRs 1-3 each containing a specific amino acid sequence. This antibody may be a CD39-neutralizing antibody. This antibody may have an ability to inhibit the growth of malignant tumor.

## Description

### Technical Field

The technical field of the present invention relates to an anti-CD39 antibody.

### Background Art

CD39 is a two-transmembrane glycoprotein and has been reported to be involved in immunosuppression of immune cells via ATP- or ADP-hydrolyzing activity (Non-Patent Literature 1).

Patent Literatures 1 and 2 describe antibodies that bind to CD39 and/or antibodies that have an ability to inhibit CD39 enzyme activity.

### Citation List

### Patent Literature

Patent Literature 1: WO 2018/167267
Patent Literature 2: Published U.S. Patent Application No. 2019/0062448

### Non-Patent Literature

Non-Patent Literature 1: Allard et al., Immunol Rev . 2017 Mar;276(1): 121-144.

### Summary of Invention

### Technical Problem

Unfortunately, the antibodies in the above Patent Literatures 1 and 2 fail to have sufficiently strong cancer cell growth inhibitory effects. Thus, there has been a room for improvement.

The purpose of the present invention is to provide, for instance, an excellent anti-CD39 antibody.

### Solution to Problem

As described in the Examples below, the present inventors immunized mice with a human CD39 recombinant antigen and produced specific anti-CD39 antibodies through screening. The effect of the obtained antibodies on cancer cells was examined, and surprisingly, they exhibited excellent cancer cell growth inhibitory effects. In particular, each antibody exerts markedly better unexpected cancer cell growth inhibitory effects than anti-CD39 antibodies prepared based on the above Patent Literatures 1 and 2.

An aspect of the present invention provides an antibody or antigen-binding fragment that binds to CD39, comprising:
(a) heavy chain CDR1 comprising an amino acid sequence set forth in SEQ ID NO: 1, heavy chain CDR2 comprising an amino acid sequence set forth in SEQ ID NO: 2, and heavy chain CDR3 comprising an amino acid sequence set forth in SEQ ID NO: 3 and light chain CDR1 comprising an amino acid sequence set forth in SEQ ID NO: 4, light chain CDR2 comprising an amino acid sequence set forth in SEQ ID NO: 5, and light chain CDR3 comprising an amino acid sequence set forth in SEQ ID NO: 6;
(b) heavy chain CDR1 comprising an amino acid sequence set forth in SEQ ID NO: 7, heavy chain CDR2 comprising an amino acid sequence set forth in SEQ ID NO: 8, and heavy chain CDR3 comprising an amino acid sequence set forth in SEQ ID NO: 9 and light chain CDR1 comprising an amino acid sequence set forth in SEQ ID NO: 10, light chain CDR2 comprising an amino acid sequence set forth in SEQ ID NO: 11, and light chain CDR3 comprising an amino acid sequence set forth in SEQ ID NO: 12;
(c) heavy chain CDR1 comprising an amino acid sequence set forth in SEQ ID NO: 13, heavy chain CDR2 comprising an amino acid sequence set forth in SEQ ID NO: 14, and heavy chain CDR3 comprising an amino acid sequence set forth in SEQ ID NO: 15 and light chain CDR1 comprising an amino acid sequence set forth in SEQ ID NO: 16, light chain CDR2 comprising an amino acid sequence set forth in SEQ ID NO: 17, and light chain CDR3 comprising an amino acid sequence set forth in SEQ ID NO: 18;
(d) heavy chain CDR1 comprising an amino acid sequence set forth in SEQ ID NO: 19, heavy chain CDR2 comprising an amino acid sequence set forth in SEQ ID NO: 20, and heavy chain CDR3 comprising an amino acid sequence set forth in SEQ ID NO: 21and light chain CDR1 comprising an amino acid sequence set forth in SEQ ID NO: 22, light chain CDR2 comprising an amino acid sequence set forth in SEQ ID NO: 23 and light chain CDR3 comprising an amino acid sequence set forth in SEQ ID NO: 24;
(e) heavy chain CDR1 comprising an amino acid sequence set forth in SEQ ID NO: 25, heavy chain CDR2 comprising an amino acid sequence set forth in SEQ ID NO: 26, and heavy chain CDR3 comprising an amino acid sequence set forth in SEQ ID NO: 27 and light chain CDR1 comprising an amino acid sequence set forth in SEQ ID NO: 28, light chain CDR2 comprising an amino acid sequence set forth in SEQ ID NO: 29 and light chain CDR3 comprising an amino acid sequence set forth in SEQ ID NO: 30;
(f) heavy chain CDR1 comprising an amino acid sequence set forth in SEQ ID NO: 31, heavy chain CDR2 comprising an amino acid sequence set forth in SEQ ID NO: 32, and heavy chain CDR3 comprising an amino acid sequence set forth in SEQ ID NO: 33 and light chain CDR1 comprising an amino acid sequence set forth in SEQ ID NO: 34, light chain CDR2 comprising an amino acid sequence set forth in SEQ ID NO: 35 and light chain CDR3 comprising an amino acid sequence set forth in SEQ ID NO: 36; or
(g) heavy chain CDR1 comprising an amino acid sequence set forth in SEQ ID NO: 37, heavy chain CDR2 comprising an amino acid sequence set forth in SEQ ID NO: 38, and heavy chain CDR3 comprising an amino acid sequence set forth in SEQ ID NO: 39 and light chain CDR1 comprising an amino acid sequence set forth in SEQ ID NO: 40, light chain CDR2 comprising an amino acid sequence set forth in SEQ ID NO: 41 and light chain CDR3 comprising an amino acid sequence set forth in SEQ ID NO: 42.

Use of this antibody or antigen-binding fragment can elicit excellent cancer cell growth inhibitory effects.

Another aspect of the present invention provides a polynucleotide or vector encoding an antibody or antigen-binding fragment comprising the heavy chain CDRs 1 to 3 and the light chain CDRs 1 to 3 of any one of (a) to (g) above. Use of this polynucleotide or vector allows for production of the above antibody. Another aspect of the present invention provides a cell comprising the above polynucleotide or vector. Use of this cell allows for production of the above antibody. Another aspect of the present invention provides a method for producing an antibody or antigen-binding fragment, comprising the step of growing the above cell. Use of this production method allows for production of the above antibody.

Another aspect of the present invention provides a composition comprising an antibody or antigen-binding fragment that binds to CD39 and comprises the heavy chain CDRs 1 to 3 and the light chain CDRs 1 to 3 of any one of (a) to (g) above. Use of this composition can elicit excellent cancer cell growth inhibitory effects or immune cell cytotoxicity-promoting effects. Another aspect of the present invention provides a pharmaceutical composition for treatment of malignant tumor, comprising an antibody or antigen-binding fragment that binds to CD39 and comprises the heavy chain CDRs 1 to 3 and the light chain CDRs 1 to 3 of any one of (a) to (g) above. Use of this composition can elicit excellent cancer cell growth inhibitory effects or immune cell cytotoxicity-promoting effects. Another aspect of the present invention provides a composition for inhibiting CD39 activity, comprising an antibody or antigen-binding fragment that binds to CD39 and comprises the heavy chain CDRs 1 to 3 and the light chain CDRs 1 to 3 of any one of (a) to (g) above. Use of this composition for inhibiting CD39 activity can elicit excellent CD39 activity-inhibiting effects. Another aspect of the present invention provides a composition for promoting immune cell TNFα production, comprising an antibody or antigen-binding fragment that binds to CD39 and comprises the heavy chain CDRs 1 to 3 and the light chain CDRs 1 to 3 of any one of (a) to (g) above. Use of this composition for promoting immune cell TNFα production can elicit excellent TNFα production-promoting effects. Another aspect of the present invention provides a composition for promoting immune cell cytotoxicity, comprising an antibody or antigen-binding fragment that binds to CD39 and comprises the heavy chain CDRs 1 to 3 and the light chain CDRs 1 to 3 of any one of (a) to (g) above. Use of this promoting composition can elicit excellent cytotoxicity-promoting effects.

Another aspect of the present invention provides a pharmaceutical composition for treatment of malignant tumor, which comprises an antibody or antigen-binding fragment that binds to CD39 and comprises the heavy chain CDRs 1 to 3 and the light chain CDRs 1 to 3 of any one of the above (a) to (g), wherein the pharmaceutical composition is used in combination therapy with a chemotherapeutic agent and the antibody or antigen-binding fragment that binds to CD39 and comprises the heavy chain CDRs 1 to 3 and the light chain CDRs 1 to 3 of any one of the above (a) to (g). This pharmaceutical composition may be used to provide excellent malignant tumor treatment. Another aspect of the present invention provides a pharmaceutical composition for treatment of malignant tumor, comprising a chemotherapeutic agent, wherein the pharmaceutical composition is used in combination therapy with the chemotherapeutic agent and an antibody or antigen-binding fragment comprising the heavy chain CDRs 1 to 3 and the light chain CDRs 1 to 3 of any one of (a) to (g) above. This pharmaceutical composition may be used to provide excellent malignant tumor treatment. Another aspect of the present invention provides a kit comprising the antibody or antigen-binding fragment. Use of this kit can elicit excellent cancer cell growth inhibitory effects. Another aspect of the present invention provides a composition for selectively removing CD39^{hi} cells, comprising an anti-CD39 antibody. Use of this composition allows for selective removal of CD39^{hi} cells.

### Brief Description of Drawings

[Figure 1] Fig. 1 shows the results of measuring CD39 inhibitory activity.
[Figure 2] Fig. 2 shows the results of measuring CD39 inhibitory activity.
[Figure 3] Fig. 3 shows the results of measuring CD39 inhibitory activity.
[Figure 4A] Fig. 4A shows the results of measuring CD39 inhibitory activity.
[Figure 4B] Fig. 4B shows the results of measuring CD39 inhibitory activity.
[Figure 5] Fig. 5 shows the results of measuring CD39 inhibitory activity.
[Figure 6] Fig. 6 is tables showing the results of SPR analysis.
[Figure 7] Fig. 7 is tables showing the results of SPR analysis.
[Figure 8] Fig. 8 shows the results of FCM analysis (human CD39).
[Figure 9] Fig. 9 shows the results of FCM analysis (human CD39).
[Figure 10] Fig. 10 shows the results of FCM analysis (human CD39).
[Figure 11A] Fig. 11A shows the results of FCM analysis (human CD39).
[Figure 11B] Fig. 11B shows the results of FCM analysis (human CD39).
[Figure 12] Fig. 12 shows the results of FCM analysis (monkey CD39).
[Figure 13] Fig. 13 shows the results of FCM analysis (monkey CD39).
[Figure 14] Fig. 14 shows the results of FCM analysis (monkey CD39).
[Figure 15] Fig. 15 shows the results of FCM analysis (monkey CD39).
[Figure 16] Fig. 16 shows the results of FCM analysis (monkey CD39).
[Figure 17] Fig. 17 shows the results of measuring CD39 inhibitory activity.
[Figure 18] Fig. 18 shows the results of FCM analysis (Treg CD39).
[Figure 19] Fig. 19 shows the results of measuring the growth of CD4-positive T cells.
[Figure 20] Fig. 20 shows the results of measuring the growth of CD8-positive T cells.
[Figure 21] Fig. 21 shows the results of measuring the growth of CD4-positive T cells.
[Figure 22] Fig. 22 shows the results of measuring the growth of CD8-positive T cells.
[Figure 23] Fig. 23 shows the results of measuring the growth of CD3-positive T cells.
[Figure 24] Fig. 24 shows the results of measuring the growth of CD4-positive T cells.
[Figure 25] Fig. 25 shows the results of measuring the growth of CD8-positive T cells.
[Figure 26] Fig. 26 shows the results of measuring the level of TNFα.
[Figure 27] Fig. 27 shows the results of measuring the concentration of antibody after exposed in blood.
[Figure 28] Fig. 28 shows the results of measuring anti-tumor activity of each antibody.
[Figure 29] Fig. 29 shows the results of measuring anti-tumor activity of each antibody.
[Figure 30] Fig. 30 shows the results of measuring anti-tumor activity of each antibody.
[Figure 31] Fig. 31 shows the results of measuring anti-tumor activity of each antibody.
[Figure 32] Fig. 32 shows the results of measuring anti-tumor activity of each antibody.
[Figure 33] Fig. 33 shows the results of measuring anti-tumor activity of each antibody *(in vivo,* single drug).
[Figure 34] Fig. 34 shows the results of measuring anti-tumor activity of each antibody *(in vivo,* used in combination).
[Figure 35] Fig. 35 shows the results of measuring the weight of heart.
[Figure 36] Fig. 36 shows the amino acid sequence of each CDR of the antibodies in Examples.
[Figure 37] Fig. 37 shows the amino acid sequence of each variable region of the antibodies in Examples.
[Figure 38] Fig. 38 shows the amino acid sequence of each variable region of the antibodies in Examples.
[Figure 39] Fig. 39 shows the amino acid sequence of each variable region of the antibodies in Examples.
[Figure 40] Fig. 40 shows the amino acid sequence of each variable region of the antibodies in Examples.
[Figure 41] Fig. 41 shows the amino acid sequence of each constant region of the antibodies in Examples.
[Figure 42] Fig. 42 is a table showing the results of SPR analysis.
[Figure 43] Fig. 43 shows the results of measuring CD39 inhibitory activity.
[Figure 44] Fig. 44 shows the results of measuring the number of CD4⁺ T cells.
[Figure 45] Fig. 45 shows the results of measuring the percentage of CD4⁺ T cells that had divided 6 times.
[Figure 46] Fig. 46 shows the results of measuring the number of CD8⁺ T cells.
[Figure 47] Fig. 47 shows the results of measuring the percentage of CD8⁺ T cells that had divided 6 times.
[Figure 48] Fig. 48 shows the results of glycan analysis.
[Figure 49] Fig. 49 shows the results of analyzing binding to activated Tregs.
[Figure 50] Fig. 50 shows the results of analyzing binding to activated Tregs.
[Figure 51] Fig. 51 shows the results of the ADCC activity assay (reporter assay).
[Figure 52] Fig. 52 shows the results of the ADCC activity assay (using a cancer cell line).
[Figure 53] Fig. 53 shows the results of SPR analysis.
[Figure 54A-C] Figure 54A-C summarize ADCC activity, affinity for FcyRIIIa, and fucosylation percentage.
[Figure 55] Fig. 55 shows the results of cell-type analysis.
[Figure 56] Fig. 56 shows the results of evaluating Treg removal.
[Figure 57] Fig. 57 shows the results of evaluating Treg removal.
[Figure 58] Fig. 58 shows the results of the study of the effects on CD8⁺T_{CM} and CD8⁺T_{EM}.
[Figure 59] Fig. 59 shows the results of the cytotoxic T cell induction test.
[Figure 60] Fig. 60 shows the results of the cancer cell growth inhibition test with Treg-removed PBMCs.
[Figure 61] Fig. 61 is a table showing the results of SPR analysis.
[Figure 62] Fig. 62 is a table showing the results of SPR analysis.
[Figure 63] Fig. 63 shows the results of measuring CD39 inhibitory activity.
[Figure 64] Fig. 64 is a table showing the results of glycan analysis.
[Figure 65] Fig. 65 shows the results of the ADCC activity assay (using Treg as a target).
[Figure 66] Fig. 66 shows the correlation between ADCC activity and FcyRIIIa-binding activity.
[Figure 67] Fig. 67 shows the correlation between ADCC activity and fucosylation percentage.
[Figure 68] Fig. 68 shows the results of the ADCC activity assay (using OAW-42 as a target).
[Figure 69] Fig. 69 shows the results of the ADCC activity assay (using OAW-42 as a target).
[Figure 70] Fig. 70 shows the results of cell-type analysis in CD4⁺ T cells.
[Figure 71] Fig. 71 shows the results of cell-type analysis in CD8⁺ T cells.
[Figure 72] Fig. 72 shows the results of cell-type analysis in CD4⁺ T cells.

### Description of Embodiments

Hereinafter, embodiments of the present invention will be described in detail. Note that repeated descriptions of the same content are omitted, if appropriate, so as to avoid redundancy.

An embodiment of the present invention provides a novel anti-CD39 antibody. This anti-CD39 antibody is, for example, an antibody comprising:
(a) heavy chain CDR1 comprising an amino acid sequence set forth in SEQ ID NO: 1, heavy chain CDR2 comprising an amino acid sequence set forth in SEQ ID NO: 2, and heavy chain CDR3 comprising an amino acid sequence set forth in SEQ ID NO: 3 and light chain CDR1 comprising an amino acid sequence set forth in SEQ ID NO: 4, light chain CDR2 comprising an amino acid sequence set forth in SEQ ID NO: 5, and light chain CDR3 comprising an amino acid sequence set forth in SEQ ID NO: 6;
(b) heavy chain CDR1 comprising an amino acid sequence set forth in SEQ ID NO: 7, heavy chain CDR2 comprising an amino acid sequence set forth in SEQ ID NO: 8, and heavy chain CDR3 comprising an amino acid sequence set forth in SEQ ID NO: 9 and light chain CDR1 comprising an amino acid sequence set forth in SEQ ID NO: 10, light chain CDR2 comprising an amino acid sequence set forth in SEQ ID NO: 11, and light chain CDR3 comprising an amino acid sequence set forth in SEQ ID NO: 12;
(c) heavy chain CDR1 comprising an amino acid sequence set forth in SEQ ID NO: 13, heavy chain CDR2 comprising an amino acid sequence set forth in SEQ ID NO: 14, and heavy chain CDR3 comprising an amino acid sequence set forth in SEQ ID NO: 15 and light chain CDR1 comprising an amino acid sequence set forth in SEQ ID NO: 16, light chain CDR2 comprising an amino acid sequence set forth in SEQ ID NO: 17, and light chain CDR3 comprising an amino acid sequence set forth in SEQ ID NO: 18;
(d) heavy chain CDR1 comprising an amino acid sequence set forth in SEQ ID NO: 19, heavy chain CDR2 comprising an amino acid sequence set forth in SEQ ID NO: 20, and heavy chain CDR3 comprising an amino acid sequence set forth in SEQ ID NO: 21and light chain CDR1 comprising an amino acid sequence set forth in SEQ ID NO: 22, light chain CDR2 comprising an amino acid sequence set forth in SEQ ID NO: 23 and light chain CDR3 comprising an amino acid sequence set forth in SEQ ID NO: 24;
(e) heavy chain CDR1 comprising an amino acid sequence set forth in SEQ ID NO: 25, heavy chain CDR2 comprising an amino acid sequence set forth in SEQ ID NO: 26, and heavy chain CDR3 comprising an amino acid sequence set forth in SEQ ID NO: 27 and light chain CDR1 comprising an amino acid sequence set forth in SEQ ID NO: 28, light chain CDR2 comprising an amino acid sequence set forth in SEQ ID NO: 29 and light chain CDR3 comprising an amino acid sequence set forth in SEQ ID NO: 30;
(f) heavy chain CDR1 comprising an amino acid sequence set forth in SEQ ID NO: 31, heavy chain CDR2 comprising an amino acid sequence set forth in SEQ ID NO: 32, and heavy chain CDR3 comprising an amino acid sequence set forth in SEQ ID NO: 33 and light chain CDR1 comprising an amino acid sequence set forth in SEQ ID NO: 34, light chain CDR2 comprising an amino acid sequence set forth in SEQ ID NO: 35 and light chain CDR3 comprising an amino acid sequence set forth in SEQ ID NO: 36; or
(g) heavy chain CDR1 comprising an amino acid sequence set forth in SEQ ID NO: 37, heavy chain CDR2 comprising an amino acid sequence set forth in SEQ ID NO: 38, and heavy chain CDR3 comprising an amino acid sequence set forth in SEQ ID NO: 39 and light chain CDR1 comprising an amino acid sequence set forth in SEQ ID NO: 40, light chain CDR2 comprising an amino acid sequence set forth in SEQ ID NO: 41 and light chain CDR3 comprising an amino acid sequence set forth in SEQ ID NO: 42.

Such an antibody can elicit excellent cancer cell growth inhibitory effects as demonstrated in the Examples below. Examples of the excellent cancer cell growth inhibitory effects include growth suppression effects higher and occurring in shorter time, higher effects of promoting TNFα production, or higher effects of releasing immunosuppression than those of conventional anti-CD39 antibodies. Examples of the excellent cancer cell growth inhibitory effects may also include effects of suppressing a heart weight loss when doxorubicin is co-administered, Treg function-reducing effects, Teff division-promoting effects, Treg lysis effects, Treg division-inhibiting effects, Treg frequency-decreasing effects, or CD39^{hi} cell selective removal effects. The inhibitory effect of anti-CD39 antibody on cancer cell growth may be caused by promoting cytotoxicity caused by immune cells (e.g., T cells or PBMCs). The promotion of cytotoxicity may be caused by inhibition of adenosine production because the anti-CD39 antibody inhibits CD39 activity. The promotion of cytotoxicity is very beneficial for cancer therapy since it causes the number of cancer cells to be actively lowered. The above antibodies are also each excellent as an antibody for the preparation of an antibody drug with enhanced cancer therapeutic effects by lowering fucosylation or an antibody drug with excellent CD39^{hi} cell-selective removal effects.

In one embodiment of the present invention, CD39 includes the protein represented by ENTPD1 or Ectonucleoside triphosphate diphosphohydrolase 1. Details of the amino acid sequence of CD39 can be obtained from, for instance, NCBI or UniProt website. The primary accession number of CD39 listed in UniProt is, for example, P49961. The amino acid sequence of human CD39 is, for instance, SEQ ID NO: 98. If CD39 has CD39 activity, the biological origin is not limited. Examples of the CD39 activity include ATPase activity, ADPase activity, or activity to convert ATP or ADP to AMP. The CD39 activity may be evaluated by incubating the cells in an environment where CD39 and ATP can be in contact and measuring an amount of decrease in ATP. The amount of decrease in ATP may be evaluated by measuring chemiluminescence using, for example, CellTiter-Glo (Promega). Examples of CD39 include CD39 derived from a human, monkey, mouse, rat, dog, or cat.

The "anti-CD39 antibody" in an embodiment of the present invention includes an antibody with an ability to inhibit CD39 activity. Examples of the inhibition of activity include inhibition of ATPase activity. The "anti-CD39 antibody" in an embodiment of the present invention includes an antibody with an ability to inhibit malignant tumor cell growth. The growth inhibition may be achieved by inhibiting the growth of malignant tumor in the presence of immune cells. In one embodiment of the present invention, the anti-CD39 antibodies include an antibody with CD39-neutralizing activity (neutralizing antibody), an antibody with an ability to inhibit CD39 functions, an antibody with an ability to promote cytotoxicity, an antibody with an ability to promote TNF-α production by immune cells, an antibody with an ability to activate immune cells, an antibody with an ability to inhibit a decrease in heart weight when doxorubicin is co-administered, an antibody with an ability to decrease Treg functions, an antibody with an ability to promote Teff division, an antibody with an ability to lyse Tregs, an antibody with an ability to inhibit Treg division, an antibody with an ability to lower the Treg frequency, or an antibody with an effect of selectively removing CD39^{hi} cells. The neutralizing antibodies include an antibody that can inhibit CD39 activity. In one embodiment of the present invention, the anti-CD39 antibodies include an antibody that induces cytotoxicity against malignant tumor cells but not against normal cells. Examples of the immune cell in an embodiment of the present invention include a T cell or PBMC. Examples of the T cell in an embodiment of the present invention include Treg (regulatory T cell). As used herein, the antibodies that inhibit CD39 activity include an antibody with a function of inhibiting CD39 activity, or an antibody with an ability to inhibit CD39 activity. As used herein, the antibodies that inhibit the growth of malignant tumor cells include an antibody with a function of inhibiting the growth of malignant tumor cells, or an antibody with an ability to inhibit the growth of malignant tumor cells.

In one embodiment of the present invention, the method for producing an anti-CD39 antibody is not particularly limited, and the antibody may be produced through, for example, step (i) of designing an antibody variable region sequence (e.g., a humanized antibody variable region sequence) containing any of the CDR sets in (a)-(g) above by using modeling/simulation software (e.g., BIOVIA Discovery Studio 2020 (Dassault Systems)), step (ii) of incorporating the antibody variable region sequence into an expression vector encoding a constant region (e.g., a human constant region), step (iii) of transfecting cells with the expression vector, or step (iv) of collecting and purifying the antibody from the cells. The anti-CD39 antibody may be produced using the grafting technology described in "Safdari et al., Biotechnol Genet Eng Rev. 2013; 29: 175-86." through the step of grafting any of the CDR sets in (a)-(g) above to an existing antibody sequence. The anti-CD39 antibody may be produced through the step of immunizing mammals with CD39 and collecting and purifying the antibody. The anti-CD39 antibody may be produced through the step of screening antibodies for their ability to inhibit CD39 activity or their strength of binding to CD39 as an indicator.

The "anti-CD39 antibody" in an embodiment of the present invention includes the form of monoclonal antibody. The monoclonal antibody could act on CD39 more efficiently than a polyclonal antibody.

An anti-CD39 antibody according to an embodiment of the present invention includes a form of antibody fragment having CD39-binding activity (hereinafter, sometimes referred to as an "antigen-binding fragment"). Examples of the antigen-binding fragment include an antigen-binding fragment having any of the CDR sets in (a)-(g) above and having CD39 binding activity.

An anti-CD39 antibody according to an embodiment of the present invention may have a K_{D} (M), for instance, 9.0 × 10⁻⁹, 5.0 × 10⁻⁹, 3.0 × 10⁻⁹, 1.0 × 10⁻⁹, 9.0 × 10⁻¹⁰, 5.0 × 10⁻¹⁰, 3.0 × 10⁻¹⁰, or 1.0 × 10⁻¹⁰ or less. K_{D} (M) may be a value measured by SPR. K_{D} (M) may be K_{D} (M) for CD39 (e.g., human CD39), or between any two of these values.

The antibody class of an anti-CD39 antibody according to an embodiment of the present invention is not particularly limited and may be, for instance, IgG, IgA, IgM, IgD, or IgE. In addition, the subclass of the antibody is not particularly limited and may be, for instance, IgG1, IgG2, IgG3, IgG4, IgA1, or IgA2.

An anti-CD39 antibody according to an embodiment of the present invention may be an antibody that binds to a wild-type CD39 or a CD39 mutant. Examples of the mutant CD39 include those having an SNP(s), etc., that caused by variation in individual DNA sequences.

In one embodiment of the present invention, CDR (complementarity determining region) is a region that forms a site of binding to its antigen. Typically, CDRs are located on the Fv (variable region: including the heavy chain variable region (VH) and light chain variable region (VL)) of the antibody. Typically, CDRs each consist of about 3 to 30 amino acid residues, CDR1, CDR2 and CDR3, on the respective heavy and light chains. It is known that the CDRs of the heavy chain contribute particularly to the binding of the antibody to the antigen. Among the CDRs, CDR3 is known to contribute the most to the binding of the antibody to the antigen. The Fv regions other than CDRs are called framework regions and include FR1, FR2, FR3 and FR4, which are relatively well conserved among antibodies (e.g., Kindt et al., Kuby Immunology, 6th ed., W.H. Freeman and Co.)

One embodiment of the present invention is an antibody that binds to CD39 and contains heavy chain CDR1, heavy chain CDR2, heavy chain CDR3, light chain CDR1, light chain CDR2, or light chain CDR3, wherein the heavy chain CDR1 contains the amino acid sequence represented by SEQ ID NO: 1, 7, 13, 19, 25, 31, or 37, the heavy chain CDR2 contains the amino acid sequence represented by SEQ ID NO: 2, 8, 14, 20, 26, 32, or 38, the heavy chain CDR3 contains the amino acid sequence represented by SEQ ID NO: 3, 9, 15, 21, 27, 33, or 39, the light chain CDR1 contains the amino acid sequence represented by SEQ ID NO: 4, 10, 16, 22, 28, 34, or 40, the light chain CDR2 contains the amino acid sequence represented by SEQ ID NO: 5, 11, 17, 23, 29, 35, or 41, and the light chain CDR3 contains the amino acid sequence represented by SEQ ID NO: 6, 12, 18, 24, 30, 36, or 42.

One embodiment of the present invention is an antibody that binds to CD39 and contains a heavy chain variable region or a light chain variable region, wherein the heavy chain variable region contains the amino acid sequence represented by SEQ ID NO: 43, 45, 47, 49, 51, 53, 55, 57, 59, 61, 63, 65, 67, 69, 71, 73, 75, 77, 79, 81, 83, 85, or 99 and the light chain variable region contains the amino acid sequence represented by SEQ ID NO: 44, 46, 48, 50, 52, 54, 56, 58, 60, 62, 64, 66, 68, 70, 72, 74, 76, 78, 80, 82, 84, 86, or 100. In one embodiment of the present invention, the anti-CD39 antibodies include (h) a heavy chain variable region and a light chain variable region containing the amino acid sequences represented by SEQ ID NOs: 43 and 44, respectively, (i) a heavy chain variable region and a light chain variable region containing the amino acid sequences represented by SEQ ID NOs: 45 and 46, respectively, (j) a heavy chain variable region and a light chain variable region containing the amino acid sequences represented by SEQ ID NOs: 47 and 48, respectively, (k) a heavy chain variable region and a light chain variable region containing the amino acid sequences represented by SEQ ID NOs: 49 and 50, respectively, (l) a heavy chain variable region and a light chain variable region containing the amino acid sequences represented by SEQ ID NOs: 51 and 52, respectively, (m) a heavy chain variable region and a light chain variable region containing the amino acid sequences represented by SEQ ID NOs: 53 and 54, respectively, (n) a heavy chain variable region and a light chain variable region containing the amino acid sequences represented by SEQ ID NOs: 55 and 56, respectively, (o) a heavy chain variable region and a light chain variable region containing the amino acid sequences represented by SEQ ID NOs: 57 and 58, respectively, (p) a heavy chain variable region and a light chain variable region containing the amino acid sequences represented by SEQ ID NOs: 59 and 60, respectively, (q) a heavy chain variable region and a light chain variable region containing the amino acid sequences represented by SEQ ID NOs: 61 and 62, respectively, (r) a heavy chain variable region and a light chain variable region containing the amino acid sequences represented by SEQ ID NOs: 63 and 64, respectively, (s) a heavy chain variable region and a light chain variable region containing the amino acid sequences represented by SEQ ID NOs: 65 and 66, respectively, (h) a heavy chain variable region and a light chain variable region containing the amino acid sequences represented by SEQ ID NOs: 67 and 68, respectively, (t) a heavy chain variable region and a light chain variable region containing the amino acid sequences represented by SEQ ID NOs: 69 and 70, respectively, (u) a heavy chain variable region and a light chain variable region containing the amino acid sequences represented by SEQ ID NOs: 71 and 72, respectively, (v) a heavy chain variable region and a light chain variable region containing the amino acid sequences represented by SEQ ID NOs: 73 and 74, respectively, (w) a heavy chain variable region and a light chain variable region containing the amino acid sequences represented by SEQ ID NOs: 75 and 76, respectively, (x) a heavy chain variable region and a light chain variable region containing the amino acid sequences represented by SEQ ID NOs: 77 and 78, respectively, (y) a heavy chain variable region and a light chain variable region containing the amino acid sequences represented by SEQ ID NOs: 79 and 80, respectively, (z) a heavy chain variable region and a light chain variable region containing the amino acid sequences represented by SEQ ID NOs: 81 and 82, respectively, (a2) a heavy chain variable region and a light chain variable region containing the amino acid sequences represented by SEQ ID NOs: 83 and 84, respectively, (b2) a heavy chain variable region and a light chain variable region containing the amino acid sequences represented by SEQ ID NOs: 85 and 86, respectively, or (c2) a heavy chain variable region and a light chain variable region containing the amino acid sequences represented by SEQ ID NOs: 99 and 100, respectively. Use of such an antibody can elicit excellent cancer cell growth inhibitory effects.

In one embodiment of the present invention, an anti-CD39 antibody includes an anti-CD39 antibody that competes with an antibody (e.g., an antibody having any of the CDR sets of (a)-(g) above) (hereinafter, sometimes referred to as a reference antibody) according to an embodiment of the present invention for binding to CD39. The competition involves the binding of an antibody to its antigen thereby inhibiting the binding of another antibody to the same antigen. The competing antibody can be checked, for example, by a competitive binding assay. Examples of the competitive binding assay include competitive ELISA or competitive FACS analysis (see, for example, Zhou et al., J Gen Virol. 2008 Feb; 89(Pt 2): 500-508.). The binding may be measured, for example, by surface plasmon resonance method. A competitive binding assay may, for example, include the steps of: coating an antigen on a microplate, adding a test antibody and incubating them to form binding between the antigen and the test antibody; adding a labeled reference antibody to wells, incubating and washing it; or quantifying the amount of binding of the labeled reference antibody to the antigen. At this time, for example, the amount of binding of the biotinylated reference antibody may be detected by measuring absorbance at the measurement wavelength of 450 nm by using HRP-conjugated streptavidin and 3,3',5,5'-tetramethylbenzidine. The inhibition rate may be evaluated as the percentage of decrease in the amount of binding of the labeled reference antibody when compared to the absence of the test antibody or the addition of a negative control. Inhibition of the reference antibody by the competing antibody includes inhibition of 20, 30, 40, 50, 60, 70, 80, 90% or more, or 100%. This inhibition is preferably 40% or higher, and particularly preferably 50% or higher. The competing antibody may bind to the epitope to which the reference antibody binds.

An antibody according to one embodiment of the present invention includes an antibody that can bind to CD39. The term "antibody" includes a molecule or population thereof that can specifically bind to a specific epitope on an antigen. In addition, the antibody may be a polyclonal or monoclonal antibody. The antibody may be present in various forms, and examples include at least one form selected from the group consisting of full-length antibodies (antibodies with Fab and Fc regions), Fv antibodies, Fab antibodies, F(ab')₂ antibodies, Fab' antibodies, diabodies, single-chain antibodies (e.g., scFv), dsFv, scFvFc, multispecific antibodies (e.g., bispecific antibodies), antigen binding peptides or polypeptides, chimeric antibodies, mouse antibodies, humanized antibodies, human antibodies, and their equivalents (or equivalents). In addition, the antibody may include a modified antibody or an intact antibody. The modified antibody may be an antibody linked to each molecule such as polyethylene glycol. The modified antibody may be obtained by chemically modifying an antibody using a known procedure. The antibody may also be a fusion protein. This fusion protein may have a polypeptide or oligopeptide (e.g., a His tag) linked to the N or C terminal of the antibody. Also, the fusion protein may be obtained by fusing a part(s) of mouse or human antibody sequence. The antibody may also be a conjugate antibody (e.g., antibody-drug conjugate). The drug may be, for example, a cytotoxic or anticancer agent. Such a modified antibody, fusion protein, or conjugate antibody is also included in a form of the antibody. The amino acid sequence, class, or subclass of the antibody may be derived from, for instance, a human or a non-human mammal (e.g., a rat, mouse, rabbit, cow, monkey). In addition, examples of the antibody include an isolated antibody, a purified antibody, or a recombinant antibody. Further, the antibody may be used, for instance, *in vitro* or *in vivo.*

As used herein, the term "monoclonal antibody" includes an antibody in the case where individual antibodies constituting a population substantially react with the identical epitope. Also, the monoclonal antibody may be an antibody in the case where individual antibodies constituting a population are substantially the same (provided that naturally occurring mutations are permitted). The method for preparing a monoclonal antibody is not particularly limited, and the monoclonal antibody may be prepared by substantially the same method as the hybridoma method described in, for instance, "Kohler G, Milstein C., Nature. 1975 Aug 7; 256(5517): 495-497". Alternatively, the monoclonal antibody may be prepared by substantially the same method as the recombinant method disclosed in U.S. Patent No. 4816567. In addition, the monoclonal antibody may be isolated from a phage antibody library by using substantially the same method as the technology described in "Clackson et al., Nature. 1991 Aug 15; 352(6336): 624-628" or "Marks et al., J Mol Biol. 1991 Dec 5; 222(3): 581-597". Further, the method described in "Protein Experiment handbook, YODOSHA CO .,LTD. (2003): 92-96" may be used for the preparation.

As used herein, the term "chimeric antibody" may be obtained by, for instance, linking variable regions of an antibody and a constant region of a xenogeneic antibody and can be prepared by gene recombinant technology. Examples include chimeric antibodies derived from non-human mammals and humans (e.g., mouse-human chimeric antibodies). The mouse/human chimeric antibody can be produced by, for instance, the method described in "Roguska et al., Proc Natl Acad Sci U S A. 1994 Feb 1; 91(3): 969-973". In a basic method for producing the mouse/human chimeric antibody, a mouse leader sequence and variable region sequences present in a cloned cDNA are linked to a sequence encoding a human antibody constant region already present in a mammalian expression vector. Alternatively, a mouse leader sequence and variable region sequences present in a cloned cDNA may be linked to a sequence encoding a human antibody constant region, and this may be integrated into a mammalian expression vector. A fragment of human antibody constant region may be a H-chain constant region or a L-chain constant region of any human antibody. For example, Cγ1, Cγ2, Cγ3, or Cγ4 for the human H-chain, and C_{λ}, or C_{κ} for the L-chain.

As used herein, the term "humanized antibody" includes an antibody that has, for instance, at least one non-human CDR and human immunoglobulin-derived framework region, and a human immunoglobulin-derived constant region, and can bind to a desired antigen. An antibody may be humanized, for example, by the procedure described in the Section 1.5 below. Various other techniques known in the art may be used (e.g., Safdari et al., Biotechnol Genet Eng Rev. 2013; 29: 175-86.). In chimeric or humanized antibodies, the heavy chain constant region may be, without limitation, for example, those containing the amino acid sequence of SEQ ID NOs: 88-91. The light chain constant region may be, without limitation, for example, those containing the amino acid sequence of SEQ ID NO: 87.

As used herein, the term "Fv antibody" refers to an antibody including an antigenrecognition site. This region comprises one heavy-chain variable domain and one light-chain variable domain that are noncovalently bonded. In this structure, three CDRs of each variable domain interact with each other to be able to form an antigen-binding site on a surface of the VH-VL dimer.

As used herein, the term "Fab antibody" refers to an antibody obtained by, for instance, treating an antibody containing Fab and Fc regions with a protease papain to give fragments, in which about a half of the H chain on the N-terminal side and the whole L chain are bonded via a disulfide bond. The Fab can be obtained by, for instance, digesting, with a protease papain, an anti-CD39 antibody containing Fab and Fc regions in the above embodiment of the present invention.

As used herein, the term "F(ab')₂ antibody" refers to an antibody obtained by, for instance, treating an antibody containing Fab and Fc regions with a protease pepsin to give fragments, in which two Fab comparable portions are included. The F(ab')₂ can be obtained by, for instance, digesting, with a protease pepsin, an anti-CD39 antibody containing Fab and Fc regions in the above embodiment of the present invention. Alternatively, the Fab' portions below may be bonded via a thioether bond or disulfide bond for the production.

As used herein, the term "Fab' antibody" refers to an antibody obtained by, for instance, cleaving a disulfide bond in the hinge region of F(ab')₂. The Fab' antibody may be obtained by, for instance, treating F(ab')₂ with a reductant dithiothreitol.

As used herein, the term "scFv antibody" includes a form of an antibody in which VH and VL are linked via a suitable peptide linker. The scFv antibody may be produced by, for instance, obtaining cDNA encoding a VH and a VL of an anti-CD39 antibody according to the above embodiment of the present invention, constructing a polynucleotide encoding the VH-peptide linker-VL, cloning the polynucleotide into a vector, and using cells for its expression.

As used herein, the term "diabody" refers to an antibody having divalent antigen-binding activities and having a form of a scFv dimer. The divalent antigen-binding activities may be the same, or one of the antigen-binding activities may be different from the other. The diabody can be produced by, for instance, constructing a polynucleotide encoding an scFv such that the length of amino acid sequence of its peptide linker is 8 residues or less, cloning the resulting polynucleotide into a vector, and using cells for its expression.

As used herein, the term "dsFv" refers to an antibody obtained by constructing a polypeptide while a cysteine residue is introduced into each of a VH or a VL and bonding, via a disulfide bond, the above cysteine residues. Where each cysteine residue is introduced may be selected based on an antibody conformation prediction in accordance with the method indicated by Reiter and colleagues (Reiter et al., Protein Eng. 1994 May; 7(5): 697-704).

As used herein, the term "scFvFc" includes a form of an antibody in which scFv and Fc regions are linked via a suitable linker. For example, a polynucleotide encoding scFv, linker, and Fc region may be incorporated into a vector and the polynucleotide-expressing cells may be used for production. The scFvFc may be produced by the procedure described in the Section 1.7 below.

As used herein, the term "antigen-binding peptide or polypeptide" refers to an antibody structured by including a VH(s) and/or a VL(s) of an antibody, or CDRs 1, 2, and/or 3 thereof. The peptide containing multiple CDRs may be bonded directly or via a suitable peptide linker(s).

The method for producing the above Fv antibody, Fab antibody, F(ab')₂ antibody, Fab' antibody, scFv antibody, diabody, dsFv antibody, scFvFc, or antigen-binding peptide or polypeptide (hereinafter, sometimes referred to as "Fv antibody, etc.") is not particularly limited. For instance, DNA encoding a region of Fv antibody, etc., in an anti-CD39 antibody according to the above embodiment of the present invention may be cloned into an expression vector and cells for its expression may be used for the production. Alternatively, a chemical synthesis process such as Fmoc method (fluorenylmethyloxycarbonyl method) or tBOC method (t-butyloxycarbonyl method) may be used for the production. Note that an antigen-binding fragment in the above embodiment of the present invention may be at least one kind of the above Fv antibody, etc.

An anti-CD39 antibody according to one embodiment of the present invention includes a glycan-modified antibody. Examples of the glycan-modified antibody include an antibody in which a glycan structure linked to the Fc region of the antibody is modified. Examples of the glycan-modified antibody include a low-fucose antibody. Examples of the low-fucose antibody include an antibody in which a glycan linked to the Fc region is modified such that the fucosylation is low. In the glycan-modified antibody, the Fc region may be, for example, IgG1. In one embodiment of the present invention, the anti-CD39 antibody includes an antibody containing an Fc region with an N-type glycan while the N-type glycan is free of core fucose.

One embodiment of the present invention is a pharmaceutical composition comprising an anti-CD39 antibody, wherein the anti-CD39 antibody has an N-type glycan and 40% or more of N-type glycan in the composition is free of core fucose. Such an pharmaceutical composition can be used to elicit excellent ADCC effects as demonstrated in the Examples below. This pharmaceutical composition can be used for the treatment of malignant tumor. This pharmaceutical composition may be administered to a patient to provide a method of treating malignant tumor. Examples of the anti-CD39 antibody include an anti-CD39 antibody (e.g., an antibody having any of the CDR sets in (a)-(g) above) according to the above embodiment of the present invention. The anti-CD39 antibody may have an N-type glycan within the Fc region. The N-type glycan includes an N-glycan or N-linked glycan. The N-type glycan includes a glycan attached to the amide portion of asparagine in the Fc region. The asparagine includes asparagine or Asn297 in the CH2 domain. The core fucose includes fucose attached through an α1-6 linkage to the GlcNAc residue at the reducing end of the N-type glycan. The glycan may be analyzed by LC/MS, for example, or by subcontracting the analysis to a service company (e.g., Sumitomo Bakelite Co., Ltd.). The percentage of fucosylation (%) may be calculated, for example, by (peak area of core fucose-containing glycan)/(peak area of total N-type glycan) × 100 in LC chromatogram. The above core fucose-free N-glycan percentage may be, for example, 40, 45, 50, 55, 60, 65, 70, 75, 80, 85, 90, 95, or 100%, and may be greater than or equal to one of these values or between any two of these values. To elicit better cancer cell growth inhibitory effects, this percentage is preferably 45% or higher, more preferably 60% or higher, still more preferably 65% or higher, and most preferably 67% or higher. To produce better CD39^{hi}CD4⁺FoxP3⁺ Treg and effector Treg removal effects, this percentage is preferably 60% or higher, more preferably 65% or higher, and still more preferably 67% or higher. To reduce the percentage of CD39^{+/-}PD1⁺Tim-3⁺ fraction and lower the degree of exhaustion during induction of antigen-specific cytotoxic T cells, the percentage is preferably 60% or higher, more preferably 65% or higher, and still more preferably 67% or higher. In another aspect, one embodiment of the present invention is a pharmaceutical composition comprising an anti-CD39 antibody, wherein the anti-CD39 antibody has an N-type glycan and 40% or more of the antibody in the composition has a core fucose-free N-type glycan. In another aspect, one embodiment of the present invention is a pharmaceutical composition comprising an anti-CD39 antibody, wherein the anti-CD39 antibody has an N-type glycan and less than 60% of N-type glycan in the composition contains core fucose. Such an pharmaceutical composition can be used to elicit excellent ADCC effects as demonstrated in the Examples below. The above core fucose-containing N-glycan percentage may be, for example, 60, 55, 50, 45, 40, 35, 30, 25, 20, 15, 10, 5, or 0%, and may be less than one of these values or between any two of these values. To elicit better cancer cell growth inhibitory effects, this percentage is preferably less than 55%, more preferably less than 40%, still more preferably less than 35%, and most preferably less than 33%. To produce better CD39^{hi}CD4⁺FoxP3⁺ Treg and effector Treg removal effects, this percentage is preferably less than 40%, more preferably less than 35%, and still more preferably less than 33%. To reduce the percentage of CD39^{+/-}PD1⁺Tim-3⁺ fraction and lower the degree of exhaustion during induction of antigen-specific cytotoxic T cells, the percentage is preferably less than 40%, more preferably less than 35%, and still more preferably less than 33%.

In one embodiment of the present invention, the heavy chain constant region of the anti-CD39 antibody may have, for example, the amino acid sequence of SEQ ID NO: 88-91, or 93. In one embodiment of the present invention, the light chain constant region of the anti-CD39 antibody may have, for example, the amino acid sequence of SEQ ID NO: 87 or 92. The heavy-chain constant region and light-chain constant region are not limited, but in the examples described below, 6-3m1 and 23-7m1 have the heavy-chain constant region and light-chain constant region represented by SEQ ID NOs: 93 and 92, P4_h1lala has the heavy-chain constant region and light-chain constant region represented by SEQ ID NOs: 89 and 87, 6-3h4_IB, 6-3h4_IG, 6-3h4_IF, 6-3h4_CB, 6-3h4_HB, 6-3h4_KB, B23-7h4_IB, B23-7h4_IG, B23-7h4_IF, B23-7h4_CB, B23-7h4_HB, and B23-7h4_KB have the heavy-chain constant region and light-chain constant region represented by SEQ ID NOs: 90 and 87, respectively, and P4_sc, T86_sc, I67_sc, Tre291_sc, mAnE67_sc, HuP4_11_sc, HuP4_41_sc, HuP4_51_sc, and HuP4_IB_sc have the heavy-chain constant region represented by the amino acid sequence of SEQ ID NO: 91.

One embodiment of the present invention is a polynucleotide or vector encoding an anti-CD39 antibody (e.g., an antibody having any of the CDR sets in (a)-(g) above) according to the above embodiment of the present invention. This polynucleotide or vector may be introduced into a cell to produce a transformant. The transformant may be a human cell or a non-human mammalian (e.g., rat, mouse guinea pig, rabbit, cow, monkey) cell. Examples of the mammalian cell include Chinese hamster ovary cells (CHO cells), monkey cell COS-7, or human embryonic kidney cells (e.g., HEK293 cells). Alternatively, the transformant may be *Escherichia coli,* yeast, or the like. The above polynucleotide or vector may be constructed to be able to express an anti-CD39 antibody. The above polynucleotide or vector may contain, for instance, elements necessary for protein expression, such as a promoter, an enhancer, a replication origin, and/or an antibiotic resistance gene.

Examples of the above vector that can be used include *E. coli* plasmids (e.g., pET-Blue), *Bacillus subtilis* plasmids (e.g., pUB110), yeast plasmids (e.g., pSH19), expression plasmids for animal cells (e.g., pA1-11, pcDNA3.1-V5/His-TOPO), bacteriophages such as λ phage, or viral vectors. The vector may be an expression vector or linear.

Examples of an available method for introducing the above polynucleotide or vector into a cell include a calcium phosphate method, lipofection, electroporation, an adenoviral method, a retroviral method, or microinjection (the revised fourth ed., New Gene Engineering Handbook, YODOSHA CO .,LTD., (2003): 152-179). Examples of an available method for producing an antibody by using a cell include a method described in "Protein Experiment handbook, YODOSHA CO .,LTD., (2003); 128-142)".

An embodiment of the present invention provides a method for producing an anti-CD39 antibody, comprising the step of growing a cell comprising the polynucleotide or vector according to the above embodiment of the present invention. The above growing step includes a culturing step. In addition, this production method may include a step of collecting the anti-CD39 antibody. Further, this production method may include a step of preparing a cell culture medium. Furthermore, this production method may include a step of purifying the anti-CD39 antibody.

For purification of an antibody in an embodiment of the present invention, it is possible to use, for instance, ammonium sulfate or ethanol precipitation, Protein A, Protein G, or gel filtration chromatography, anion or cation exchange chromatography, phosphocellulose chromatography, hydrophobic interaction chromatography, affinity chromatography, hydroxyapatite chromatography, or lectin chromatography (Protein Experiment handbook, YODOSHA CO .,LTD., (2003): 27-52).

One embodiment of the present invention is a composition comprising an anti-CD39 antibody (e.g., an antibody having any of the CDR sets in (a)-(g) above) according to the above embodiment of the present invention. Use of this composition allows for efficient detection of CD39. In addition, it is possible to elicit excellent cancer cell growth inhibitory effects. Components included in this composition are not particularly limited and may include, for instance, a buffer. At least one of the below-described embodiments (e.g., optionally including a carrier) regarding inhibitors and/or pharmaceutical compositions is applicable to this composition. Examples of the composition include a composition for CD39 activity inhibition, CD39 detection, growth inhibition of malignant tumor cells, immune cell activation, promotion of TNFα production by immune cells, Treg function inhibition, Teff division promotion, Treg division inhibition, Treg frequency reduction, or CD39^{hi} cell selective removal. One embodiment of the present invention is a method for CD39 activity inhibition, CD39 detection, growth inhibition of malignant tumor cells, immune cell activation, promotion of TNFα production by immune cells, Treg function inhibition, Teff division promotion, Treg division inhibition, Treg frequency reduction, or CD39^{hi} cell selective removal, the method comprising the step of bringing CD39 (or CD39-expressing cells) into contact with an anti-CD39 antibody (an antibody having any of the CDR sets of (a)-(g) above) according to the above embodiment of the present invention. The occurrence of immune cell activation may be assessed, for example, by an increase in the growth rate of immune cells, an increase in the cytotoxicity of immune cells against cancer cells, or an increase in cytokine secretion from immune cells. The CD39-expressing cells include, for example, immune cells or malignant tumor cells that express CD39.

One embodiment of the present invention is a pharmaceutical composition comprising an anti-CD39 antibody (e.g., an antibody having any of the CDR sets in (a)-(g) above) according to the above embodiment of the present invention. This pharmaceutical composition may contains at least one pharmaceutically acceptable carrier. Examples of the pharmaceutical composition include a pharmaceutical composition for treatment of malignant tumor. Examples of the malignant tumor include CD39-positive malignant tumor. An embodiment of the present invention provides a method for treating a disease, comprising the step of administering, to a patient, the anti-CD39 antibody (or a pharmaceutical composition comprising the anti-CD39 antibody) according to the above embodiment of the present invention. Examples of the treatment include treatment of malignant tumor or cancer immunotherapy. An embodiment of the present invention provides use of the anti-CD39 antibody according to the above embodiment of the present invention for the manufacture of a pharmaceutical composition.

One embodiment of the present invention is a kit comprising an anti-CD39 antibody (e.g., an antibody having any of the CDR sets in (a)-(g) above) according to the above embodiment of the present invention. Use of this kit can elicit excellent cancer cell growth inhibitory effects. Examples of the kit include a kit for treatment of malignant tumor, CD39 activity inhibition, CD39 detection, growth inhibition of malignant tumor cells, immune cell activation, promotion of TNFα production by immune cells, Treg function inhibition, Teff division promotion, Treg division inhibition, Treg frequency reduction, or CD39^{hi} cell selective removal. This kit may contain, for example, a package insert, a buffer solution, a container, or packaging.

The "treatment" in an embodiment of the present invention involves optionally exerting an effect of ameliorating, suppressing, or preventing a disease of a patient or at least one symptom accompanied by the disease. The "pharmaceutical composition" in an embodiment of the present invention may be produce by any procedure known in the art of formulation, which procedure includes mixing, for instance, an active ingredient and at least one pharmaceutically acceptable carrier. In addition, a dosage form of the pharmaceutical composition is not limited as long as it can be used for treatment, and may be an active ingredient alone or may be a mixture of an active ingredient and any component(s). Also, the form of the above carrier is not particularly limited, and may be, for instance, a solid or liquid (e.g., a buffer). The content of the above carrier may be, for instance, a pharmaceutically effective amount. The effective amount may be, for instance, an amount sufficient to pharmaceutically stabilize or deliver the active ingredient. For instance, the buffer is effective in stabilization of the active ingredient in a vial. The dose, dosing interval, administration method, and/or administration route are not particularly limited, and may be selected, if appropriate, in view of the age, body weight, symptom, and/or affected organ of a patient. It is also preferable to contain a therapeutically effective amount or effective amount of the active ingredient so as to elicit a desired action.

In one embodiment of the present invention, examples of the subject (including a patient) include a human or a non-human mammal (e.g., at least one species of a mouse, a guinea pig, a hamster, a rat, a mouse, a rabbit, a pig, sheep, a goat, a cow, a horse, a cat, a dog, a marmoset, a monkey, or a chimpanzee). Meanwhile, the patient may also be a patient diagnosed as having malignant tumor or in need of treatment.

The malignant tumor cell growth effects may be evaluated, for example, by observing changes in malignant tumor cell growth over time after antibody administration. The state of inhibition of cell growth includes the state of a significant decrease in the growth rate of the test cells when compared to that before the antibody treatment. The growth rate may be measured, for example, by absorbance as an indicator, or it may be determined from image data. The therapeutic effect on malignant tumor may also be evaluated, for example, by observing a decrease in tumor weight after antibody administration. If the tumor weight is significantly lower than that before the antibody administration or the negative control administration, it may be judged that the treatment is effective. The therapeutic effect on malignant tumor may be measured, for example, by the level of each malignant tumor marker in a patient or patientderived sample. If the marker level is significantly lower than that before the antibody administration or the negative control administration, it may be judged that the treatment is effective. The tumor weight or marker level after antibody administration may be 0.9, 0.7, 0.5, 0.3, or 0.1 or less times that before the administration or the negative control administration. Treatment efficacy may be evaluated by imaging.

Examples of the malignant tumor in one embodiment of the present invention include tumor caused by mutations in normal cells. The malignant tumor may be derived from any organ or tissue in the body. Examples of the malignant tumor include one or more selected from the group consisting of lung cancer, esophageal cancer, stomach cancer, liver cancer, pancreas cancer, kidney cancer, adrenal gland cancer, biliary tract cancer, breast cancer, colon cancer, small intestine cancer, ovary cancer, uterus cancer, bladder cancer, prostate cancer, ureter cancer, renal pelvis cancer, ureter cancer, penis cancer, testicular cancer, brain tumor, central nervous system cancer, peripheral nervous system cancer, head and neck cancer, glioma, glioblastoma multiforme, skin cancer, melanoma, thyroid cancer, salivary gland cancer, malignant lymphoma, carcinoma, sarcoma, myeloma, and hematologic malignancies. Examples of the malignant tumor include CD39-positive malignant tumor.

One embodiment of the present invention is a pharmaceutical composition containing an anti-CD39 antibody, wherein the pharmaceutical composition is used in combination therapy with a chemotherapeutic agent and the anti-CD39 antibody. This pharmaceutical composition may be used to treat malignant tumor. In addition, the heart weight loss caused by the chemotherapeutic agent can be suppressed. One embodiment of the present invention is a pharmaceutical composition containing an anti-CD39 antibody for administration to a patient who has received a chemotherapeutic agent. One embodiment of the present invention is a pharmaceutical composition containing an anti-CD39 antibody and a chemotherapeutic agent. One embodiment of the present invention is a pharmaceutical composition containing a chemotherapeutic agent, wherein the pharmaceutical composition is used in combination therapy with the chemotherapeutic agent and an anti-CD39 antibody. One embodiment of the present invention is a pharmaceutical composition containing a chemotherapeutic agent for administration to a patient who has received an anti-CD39 antibody. One embodiment of the present invention is a method for treating a malignant tumor, comprising the steps of administering an anti-CD39 antibody to a patient, and administering a chemotherapeutic agent to the patient. In this administration method, the anti-CD39 antibody and the chemotherapeutic agent may be administered simultaneously or sequentially (in any order). In one embodiment of the present invention, the "combination" includes a form in which the anti-CD39 antibody and the chemotherapeutic agent are administered simultaneously or separately (in any order), or as a combination of the two. In addition, one embodiment of the present invention is use of an anti-CD39 antibody for the manufacture of a pharmaceutical composition used in combination therapy with a chemotherapeutic agent and the anti-CD39 antibody. In addition, one embodiment of the present invention is use of a chemotherapeutic agent for the manufacture of a pharmaceutical composition used in combination therapy with the chemotherapeutic agent and an anti-CD39 antibody.

In one embodiment of the present invention, the chemotherapeutic agent is not particularly limited and examples include an anticancer agent. Examples of the anticancer agent include a microtubule inhibitor, a DNA synthesis inhibitor, a growth factor inhibitor, a tyrosine kinase inhibitor, or a cytotoxic agent. Examples of the microtubule inhibitor include a vinca alkaloid-based agent or a taxane-based agent. Examples of the vinca alkaloid-based agent include vincristine, vinblastine, vindesine, vinorelbine, or eribulin. Examples of the taxane-based agent include paclitaxel or docetaxel. Examples of the DNA synthesis inhibitor include an antimetabolite, a topoisomerase inhibitor, a platinum preparation, an antitumor antibiotic, or an alkylating agent. Examples of the antimetabolite include pemetrexed, 5-fluorouracil, S-1, gemcitabine, or capecitabine. Examples of the topoisomerase inhibitor include irinotecan, nogitecan, etoposide, or sobuzoxane. Examples of the platinum preparation include cisplatin, oxaliplatin, nedaplatin, or carboplatin. Examples of the antitumor antibiotic include an anthracycline-based agent (e.g., doxorubicin, liposomal doxorubicin, daunorubicin, epirubicin, idarubicin, aclarubicin, amrubicin, mitoxantrone, pirarubicin), mitomycin C, actinomycin D, bleomycin, peplomycin, or zinostatin stimalamer. Examples of the alkylating agent include cyclophosphamide, dacarbazine, or ifosfamide. Examples of the growth factor inhibitor include an inhibitor of EGF, VEGF, FGF, or IGF. Examples of the growth factor inhibitor include bevacizumab, cetuximab, or panitumumab. Examples of the tyrosine kinase inhibitor include gefitinib or erlotinib. Examples of the cytotoxic agent include saporin, emtansine, deruxtecan, or vedotin. Examples of the form of chemotherapeutic agent include a low-molecular-weight compound or a high-molecular-weight compound. The chemotherapeutic agent includes a salt of any of at least one compound listed herein. In one embodiment of the present invention, examples of the form of the compound or salt include a form of solvate thereof.

The salt in one embodiment of the present invention is not particularly limited and examples include an inorganic or organic salt (see, for example, "Bharate et al., Drug Discov Today. 2021 Feb; 26(2): 384-398" or "Berge et al., J Pharm Sci. 1977 Jan; 66(1): 1-19"). Examples of the salt include a metal salt, an ammonium salt, a salt of organic base, a salt of inorganic acid, a salt of organic acid, or a salt of basic or acidic amino acid. Examples of the metal salt include an alkali metal salt (e.g., a sodium salt, a potassium salt), an alkaline earth metal salt (e.g., a calcium salt, a magnesium salt, a barium salt), or an aluminum salt. Examples of the salt of organic base include a salt of trimethylamine, triethylamine, pyridine, picoline, 2,6-lutidine, ethanolamine, diethanolamine, triethanolamine, cyclohexylamine, dicyclohexylamine, or N,N'-dibenzylethylenediamine. Examples of the salt of inorganic acid include a salt of hydrochloric acid, hydrobromic acid, nitric acid, sulfuric acid, or phosphoric acid. Examples of the salt of organic acid include a salt of formic acid, acetic acid, trifluoroacetic acid, phthalic acid, fumaric acid, mesylic acid, tosylic acid, oxalic acid, tartaric acid, maleic acid, citric acid, succinic acid, malic acid, methanesulfonic acid, benzenesulfonic acid, or p-toluenesulfonic acid. Examples of the salt of basic amino acid include a salt of arginine, lysine, or ornithine. Examples of the salt of acidic amino acid include a salt of aspartic acid or glutamic acid. Examples of the salt include a pharmaceutically acceptable salt. In one embodiment of the present invention, the wording "pharmaceutically acceptable" includes a form that has reasonable benefit for pharmaceutical use. Examples of the salt of doxorubicin in one embodiment of the present invention include doxorubicin hydrochloride. Examples of the solvate in one embodiment of the present invention include a form of compound formed using a solute and a solvent (see, for example, Healy et al., Adv Drug Deliv Rev. 2017 Aug 1;117: 25-46). Examples of the solvate include, but are not particularly limited to, a hydrate (e.g., a monohydrate, a dihydrate, a trihydrate) or an organic solvate (e.g., a solvate of alcohol (e.g., methanol, ethanol, propanol), acetone, dimethylformamide, or ethyl acetate). Examples of the solvent include a solvent that can be used to substantially maintain the physiological activity of the solute after forming the solvate.

One embodiment of the present invention is a method for promoting an ability of a composition to inhibit malignant tumor cell growth, comprising the step of increasing, in the composition, a percentage of an anti-CD39 antibody (e.g., an antibody having any of the CDR sets in (a)-(g) above) according to the above embodiment of the present invention. One embodiment of the present invention is a composition comprising an anti-CD39 antibody, wherein 90% or more of the anti-CD39 antibody in the composition is an anti-CD39 (e.g., an antibody having any of the CDR sets in (a)-(g) above) according to the above embodiment of the present invention. One embodiment of the present invention is an antibody population comprising an anti-CD39 antibody, wherein 90% or more of the anti-CD39 antibody in the antibody population is an anti-CD39 (e.g., an antibody having any of the CDR sets in (a)-(g) above) according to the above embodiment of the present invention. The wording "90% or more" may mean, for instance, 90, 95, 96, 97, 98, 99% or higher, or 100% or a number between any two thereof.

One embodiment of the present invention is an antibody capable of binding to CD39, the antibody having the amino acid sequences of heavy chain and light chain CDR1-3, as defined in the definition of IMGT (Lefranc et al., Dev Comp Immunol. 2003 Jan;27(1):55-77.), the definition of Kabat (Sequences of Proteins of Immunological Interest, 5th ed. Bethesda, MD. (1991)), or Chothia's definition (Chothia et al., J. Mol. Biol., 1987; 196:901-917), in the amino acid sequence of 6-3m1, 23-7m1, P4_sc, T86_sc, I67_sc, Tre291_sc, or mAnE67_sc. Use of this antibody can elicit excellent cancer cell growth inhibitory effects. In one embodiment of the present invention, the CDR(s) is defined by IMGT or Kabat's definition, unless otherwise specified.

One embodiment of the present invention is an anti-CD39 antibody having a higher affinity for CD39, a higher malignant tumor cell growth inhibitory effect, a higher growth inhibitory effect occurring in a shorter time, a higher TNFα production-promoting effect, or a higher immunosuppression-releasing effect than the anti-CD39 antibody containing the heavy chain variable region and light chain variable region having the amino acid sequences represented by SEQ ID NOs: 94 and 95, respectively, or the anti-CD39 antibody having the heavy chain variable region and light chain variable region having the amino acid sequences represented by SEQ ID NOs: 96 and 97, respectively.

An embodiment of the present invention is a method of measuring cytotoxicity against malignant tumor cells, comprising the step of: bringing malignant tumor cells collected from a subject into contact *in vitro* with an anti-CD39 antibody (e.g., an antibody having any of the CDR sets in (a)-(g) above) according to the above embodiment of the present invention; measuring whether ATP metabolism of the malignant tumor cells is decreased under culture conditions; measuring whether cell viability is decreased; or measuring whether secretion of an immune-activating substance is increased. An embodiment of the present invention is a method of measuring cytotoxicity against malignant tumor cells, comprising the steps of: bringing malignant tumor cells collected from a subject into contact with an anti-CD39 antibody (e.g., an antibody having any of the CDR sets in (a)-(g) above) according to the above embodiment of the present invention in presence of T cells of the subject; measuring an amount of secretion of an immune-activating substance from the T cells under culture conditions. Examples of the Immune-activating substance include TNFα or IFN-γ.

One embodiment of the present invention is a method for producing a glycan-modified anti-CD39 antibody. This production method may, for example, use a fucosylation-lowering technique for the Fc region-attached glycan. This method for producing a glycan-modified antibody may include the step of culturing anti-CD39 antibody-expressing cells in presence of a fucosylation inhibitor. This production method may comprise the steps of: transfecting cells with an expression vector encoding an anti-CD39 antibody; culturing the cells in presence of a fucosylation inhibitor; or collecting the antibody from a culture supernatant after the culturing. In the above culturing, the number of culturing days from transfection of the vector into the cells to collection of the antibody may be, for example, 3, 5, 7, 9, 10, 11, 13, 14, 16, or 20 days, or more than one of those days, or within the range of any two of those days.

One embodiment of the present invention is a composition containing an anti-CD39 antibody for CD39^{hi} cell removal. Use of this composition allows for removal of CD39^{hi} cells from a subject or a cell population. The cell population may be, for example, a population of immune cells (e.g., T cells (e.g., CD4⁺ T cells or CD8⁺ T cells)). This composition includes a composition for selectively removing CD39^{hi} cells. The term "selective(ly)" includes selective for CD39^{hi} cells (e.g., preferred) over CD39^{int} cells. This composition has low side effects (e.g. toxicity) when administered *in vivo* because CD39^{hi} cells are selectively removed. The percentage of CD39^{hi} cells removed by this composition can be a higher percentage (e.g., 1.5, 2, 3 or 5 times higher) than the percentage of CD39^{int} cells removed. The cell population may be in a form of tissue. The subject may be, for example, a subject in need of CD39^{hi} cell removal (including selective removal), a subject in need of reducing side effects of anti-CD39 antibody administration, or a subject in need of treatment of malignant tumor. The CD39^{hi} cells and CD39^{int} cells may be cells expressing CD39 (e.g. CD39-positive cells). The CD39^{hi} cells may be, for example, cells that express 10,000 CD39 molecules or more per cell. The CD39^{int}cells may be, for example, cells that express less than 10,000 CD39 molecules per cell. One embodiment of the present invention, in another aspect, is a method of CD39^{hi} cell removal (including a selective removal method) using an anti-CD39 antibody. This method may include, for example, the step of bringing a cell population into contact with an anti-CD39 antibody (e.g., including the step of administering an anti-CD39 antibody to a subject). One embodiment of the present invention, in another aspect, is use of an anti-CD39 antibody for the manufacture of a composition for CD39^{hi} cell removal (including selective removal). As used herein, the CD39^{hi} cells may include, for example, CD39^{hi} immune cells (e.g. CD39^{hi} T cells). As used herein, the CD39^{int} cells may include, for example, CD39^{int} immune cells (e.g. CD39^{int} T cells).

In one embodiment of the present invention, the "amino acid" is a general term for any organic compound having an amino group and a carboxyl group. When an antibody according to an embodiment of the present invention contains a "specific amino acid sequence", any of amino acids in the amino acid sequence may be chemically modified. In addition, any of amino acids in the amino acid sequence may form a salt or a solvate. In addition, any of amino acids in the amino acid sequence may be in an L-form or D-form. In such cases, an antibody according to an embodiment of the present invention can be said to contain the above "specific amino acid sequence". Examples of the *in vivo* chemical modification of amino acids included in a protein include N-terminal modification (e.g., acetylation, myristoylation), C-terminal modification (e.g., amidation, glycosylphosphatidylinositol addition), or side-chain modification (e.g., phosphorylation, glycosylation).

As used herein, the "binding" may be mediated by either a covalent bond or noncovalent bond, and may involve, for instance, an ion bond, a hydrogen bond, a hydrophobic interaction, or a hydrophilic interaction.

As used herein, the term "significant(ly)" may mean a state of p < 0.05 or p < 0.01 after a statistically significant difference is applied in a Student's t-test (one- or two-tailed) for evaluation. Alternatively, the term may refer to a state where a substantial difference occurs.

As used herein, (a), (b), (c), (d), (e), (f), or (g) is synonymous with one or more selected from the group consisting of (a) to (g).

All the literatures cited herein are incorporated by reference in their entirety. As used herein, the term "or" is used when "at least one" matter listed in the text is acceptable. The same applies to "or". When the wording "number between any two" is indicated herein, this range encompasses the two numbers inclusive. The wording "from A to B" herein means A or more and B or less.

Hereinabove, embodiments of the present invention have been described. However, they are examples of the present invention. Hence, various configurations other than the above can be adopted. In addition, the configurations described in the above embodiments may be combined and adopted.

### Examples

Hereinbelow, the present invention will be described in more detail with reference to Examples. However, the present invention is not limited to them.

### 1.1 To isolate and culture single B cells

Mice (BALB/c, female, 7-week-old, CLEA Japan, Inc.) were each administered intraperitoneally with 10 µg or 40 µg of human CD39 recombinant antigen (SEQ ID NO: 98; self-prepared) every 2 weeks for 3 or 4 times. Approximately one week after the last administration, blood was collected from the tail vein, and the spleen of each mouse was collected after the increase of CD39-specific antibody titer in the blood was checked by antigenimmobilized ELISA.

Single cell suspension of splenocytes was prepared from the spleen and IgG-positive B cells were purified using a Memory B Cell Isolation Kit, mouse (Miltenyi Biotec, 130-095-838). The purified B cells were labeled with APC-anti-mouse IgG1 and APC-anti-mouse IgG2a supplied in the kit. In addition, the cells were stained with PE-human CD39 (autologous preparation), PE/Cy7-anti-mouse/human CD45R/B220 (BioLegend, 103222), and BV421-anti-mouse IgM (BioLegend, 406518). Using FACS Aria III (BD), a population of IgM-negative, IgG-positive, B220-positive, and human CD39-binding cells from the above stained cells was sorted one cell at a time in 150 µl medium onto a 96 well plate. The medium used was 40% FBS (Equitech-Bio, SFBM30-0500)-containing RPMI-1640 (Wako Pure Chemicals, 189-02025) supplemented with 1% penicillin-streptomycin (Nacalai Tesque, 09367-34). After incubation at 37°C, 5% CO₂ for 1 hour, 100 µl of of 3 to 5 × 10⁴ feeder cells suspended in RPMI-1640 with cytokines were added to wells of a 96 well plate and incubated at 37°C and 5% CO₂ for 12 to 14 days. The feeder cells used were from cryopreserved mouse CD40L-expressing 3T3 cells that had been irradiated with 50 Gy of X-ray. The cytokines were added at final concentrations of 10 ng/mL Mouse BAFF (ACRO Biosystems, BAF-M521y-50ug), 10 ng/mL Mouse IL-1β (BioLegend, 575104), 10 ng/mL Murine IL-4 (PeproTech, 214-14), 10 ng/mL Murine IL-6 (R&D, 214-16), 50 ng/mL Murine IL-21 10 ng/mL Murine IL-6 (PeproTech, 214-16), 10 ng/mL Mouse IL-9 (R&D, 409-ML-050), 50 ng/mL Murine IL-21 (R&D, 594-ML-100), and 10 ng/mL Mouse TNF-α (BioLegend, 575204).

### 1.2 CD39 function inhibition screening using B cell culture supernatant

Two thousand SK-MEL-28 cells (ATCC, HTB-72), a CD39^{hi}-expressing line, suspended in 50 µl EMEM (Fujifilm, 055-08975) were seeded onto a 96-well V-bottom plate and incubated at 37°C for 30 minutes with 150 µl of culture supernatant of single B cells. The cells were washed once with DPBS (Nacalai Tesque, 14249-24), then 100 µl of 5 µM ATP was added per well, and the mixture was incubated at 20°C for 30 minutes. After centrifugation, 50 µl of the supernatant was separated, 50 µl CellTiter-Glo (Promega, G9243) was added and stirred, and after incubation at room temperature for 10 min, the amount of ATP was measured by chemiluminescence while using a microplate reader. The ability of B cell culture supernatant to inhibit CD39-mediated ATP hydrolysis was evaluated based on the amount of residual ATP.

### 1.3 To amplify and isolate antibody gene sequence

The genes corresponding to the heavy and light chain variable regions of the antibody were amplified by RT-PCR for the wells in which ATP hydrolysis was inhibited by the CD39 function inhibition screening. Based on the information in the known literatures, primers used for the amplification had the sequences containing the 5'-end and 3'-end regions of each of a heavy- or light-chain variable region and mixed primers with the multiple sequences were used in view of the diversity of antibody gene sequences (heavy-chain: H1 or H2 mix, light-chain: k mix) (Lotta von Boehmer et al., Nature Protocols, vol 11, p.1908-1923 (2016)). After amplification of the antibody gene, a linker sequence for insertion into an antibody expression vector was added to each end of the gene by PCR and the resulting gene was inserted into a vector for heavy chain or light chain expression (CMV promoter, secretory signal, heavy chain variable region or light chain variable region, and heavy chain constant region (SEQ ID NO: 93) or light chain constant region (SEQ ID NO: 92), which were linked in tandem, respectively) by ligation reaction. The ligation product was transformed into *E. coli* and the plasmid for antibody expression was recovered.

### 1.4 To prepare antibody and to determine antibody gene sequence

Heavy chain expression plasmids (derived from H1 or H2 mix amplification products) and light chain expression plasmids (derived from k mix amplification products) obtained by amplification and isolation of antibody gene sequences were transfected into CHO cells. Antibodies produced in the culture supernatant from the CHO cells were collected and the CD39 inhibitory activity of bulk antibodies was checked.

*E. coli* were transformed with plasmids of the bulk antibodies with confirmed CD39 inhibitory activity, and single colonies with each heavy chain or light chain were collected from the inoculated plates, and each plasmid DNA was obtained. The corresponding heavy-chain and light-chain expression plasmids derived from the single colonies were co-transfected into CHO cells, and each antibody produced in the culture supernatant from the CHO cells was collected. This antibody was used as a single antibody for the production of the humanized antibody described in the Section 1.5 below and for the measurement of CD39 enzyme inhibitory activity described in Section 1.8 below.

The gene sequences of the variable regions of each heavy-chain or light-chain expression plasmid derived from single colonies were analyzed by Sanger sequencing, and the amino acid sequences were determined based on the gene sequences.

### 1.5 To produce humanized antibody

Humanized antibodies were produced from the antibodies obtained in the above experiments by the following procedure. Mouse antibody sequences were modified for the production of humanized antibodies by using the biological software Discovery Studio 2020 (Dassault Systems). Multiple humanized antibody variable region sequences were synthesized from a single mouse antibody variable region sequence by changing variables in the software. The humanized antibody variable region sequence prepared was incorporated into a plasmid containing the human IgG4 constant region (SEQ ID NO: 90) and the human light chain constant region (SEQ ID NO: 87) to construct a humanized antibody expression vector. The expression vector constructed was transfected into CHO cells, and the antibody produced in the culture supernatant from the CHO cells was collected and purified as follows.

### 1.6 To purify antibody

The culture supernatant of transfectant CHO cells was collected and the antibody in the culture supernatant was purified using a MonoSpin ProG or MonoSpin ProA column (GL Science, 7510-11311 or 7510-11310) or using a MonoSpin L ProA column (GL Science, 7510-11314) according to the instructions provided.

### 1.7 Antibody cloning by phage display method

Mice (BALB/c or MRL/lpr, female, 7- to 8-week-old, CLEA Japan, Inc.) were each administered intraperitoneally with 10 µg to 40 µg of human CD39 recombinant antigen (self-prepared) every 1 or 2 weeks for 3 or 4 times. Approximately one week after the last administration, blood was collected from the tail vein, and the spleen of each mouse was collected after the increase of CD39-specific antibody titer in the blood was checked by antigenimmobilized ELISA.

Total RNA was extracted from the spleens or lymph nodes of the immunized mice, converted to cDNA, and the antibody gene sequences (V_{H} and Y_{κ}) were amplified by PCR. V_{H} and Y_{κ} were linked by a linker to form a single chain Fv (scFv) and inserted into a phagemid derived from pTZ19R. The scFv-inserted phagemid was transformed into *E. coli* (strain DH12S), which was further infected with a helper phage (M13KO7) to construct a scFv phage library (size: about 1 × 10⁸ variants).

The scFv antibody phage library was subjected to panning with recombinant human CD39, and after panning, each phagemid was extracted and the scFv portion was amplified by PCR and transferred to the scFv-secreting expression vector. Since *E. coli* transformed with the scFv-secreting expression vector secretes scFv into the culture supernatant upon IPTG induction, we screened the library for scFv clones that inhibited CD39 activity by using this culture supernatant. The scFvs that were positive in the screening were sequenced. The scFvFc with the scFv and human Fc region (SEQ ID NO: 91; Fc silent) linked via a linker was expressed in ExpiCHO cells (Thermo Fisher Scientific) and was affinity-purified using a Protein A column. Each obtained antibody was used for measuring the activity to inhibit CD39 enzyme by the same procedure as described in the Section 1.8 above. In addition, humanized antibodies were produced by the same procedure as described in the Section 1.5 above (however, as before humanization, the human Fc region (SEQ ID NO: 91, Fc silent) was used for the heavy chain constant region, and the form was designed to be scFvFc). Each obtained antibody was used for measuring the activity to inhibit CD39 enzyme by the same procedure as described in the Section 1.8 above.

### 1.8 To measure activity to inhibit CD39 enzyme

The antibodies obtained in the above experiments were used to measure activity to inhibit CD39 enzyme by using the following procedure. Here, 5,000 to 6,250 cells of CD39^{hi}-expressing human melanoma cell line SK-MEL-28 (ATCC, HTB-72) or human ovarian adenocarcinoma cell line OAW-42 (CLS, 300304) were suspended in 50 µl EMEM and seeded per well in a 96-well V-bottom plate, then 50 µl antibody solution (final concentration 0.001 to 100 µg/ml) was added and the mixture was incubated at 37°C for 1 hour. Next, 50 µl ATP (final concentration 10 µM) was added and the mixture was incubated at 37°C for 30 minutes. After centrifugation, 50 µl of the supernatant was separated, 50 µl CellTiter-Glo was added and stirred, and after incubation at room temperature for 10 minutes, the level of chemiluminescence was measured using a microplate reader. Based on the amount of ATP consumed by SK-MEL-28 or OAW-42 cells, the ability of the antibody to inhibit CD39-mediated ATP hydrolysis was evaluated. POM1 is a low-molecular-weight inhibitor of E-NTPDases and was used as a positive control for human CD39 inhibition in this experiment.

### 1.9 Results

### 1.9.1 Screening for mouse antibodies

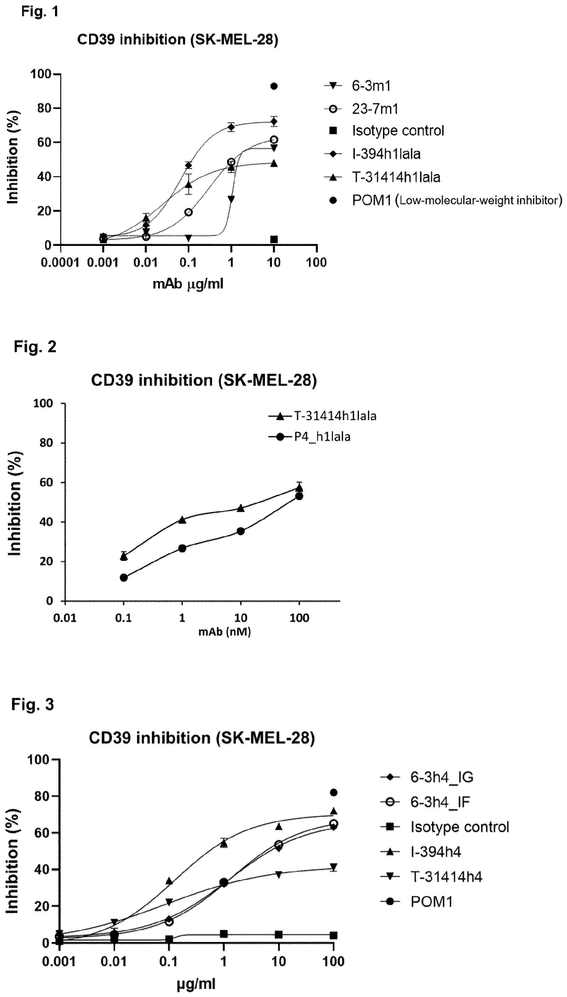

Several antibody clones with an ability to inhibit human CD39 were obtained through the experiments described in the Sections 1.1 to 1.4 above. Among the clones obtained, 6-3m1 and 23-7m1 exhibited high inhibitory activity. Fig. 1 shows the results of measuring the activity of 6-3m1 or 23-7m1 to inhibit CD39. In all figures, isotype controls are an IgG4 antibody or commercially available isotype antibody against the scorpion toxic peptide CN2 (provided that the cases where the name of the antibody is indicated are excluded). In all figures, I-394 is an anti-CD39 antibody having the amino acid sequences of the heavy chain variable region and light chain variable region of I-394 (SEQ ID NOs: 94 and 95, respectively) described in WO2018/167267. In all figures, T-31414 is an anti-CD39 antibody having the amino acid sequences of the heavy chain variable region and light chain variable region of I-31414 (SEQ ID NOs: 96 and 97, respectively) described in US2019/0062448. The heavy-chain constant region of I-394 and T-31414 has the amino acid sequence of SEQ ID NO: 89 for those whose name ends in h1lala or SEQ ID NO: 90 for those whose name ends in h4, and the light-chain constant region of both has the amino acid sequence of SEQ ID NO: 87.

### 1.9.2 Screening by phage display method

By the experiment described in the Section 1.7 above, P4_sc, T86_sc, I67_sc, Tre291_sc, and mAnE67_sc were obtained as antibodies with an activity to inhibit human CD39. In addition, P4_h1lala was produced, which is a chimeric antibody with V_{H} and Y_{κ} of P4_sc, human IgG1-lala (SEQ ID NO: 89, Fc silent), and human light chain constant region (SEQ ID NO: 87). Fig. 2 shows the results of measuring the activity of P4_h1lala to inhibit CD39.

### 1.9.3 To evaluate humanized antibody

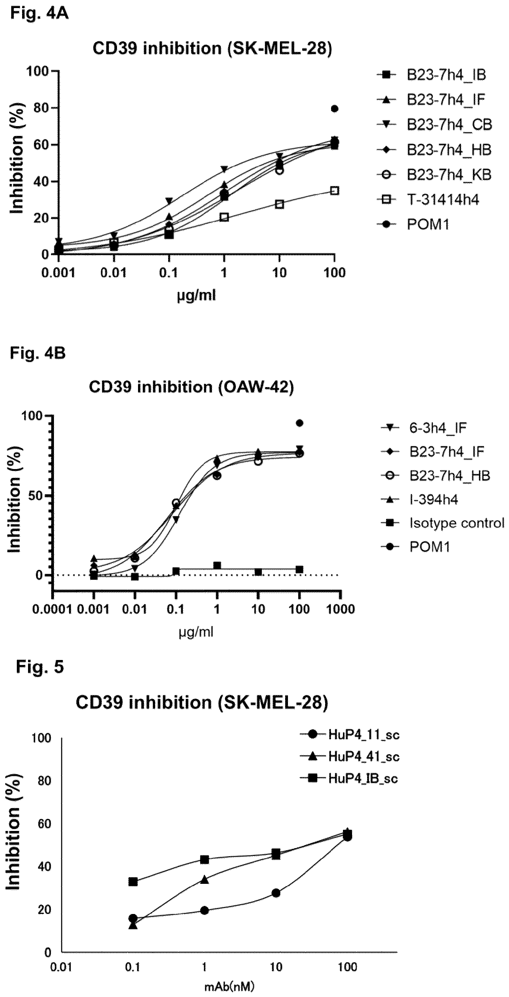

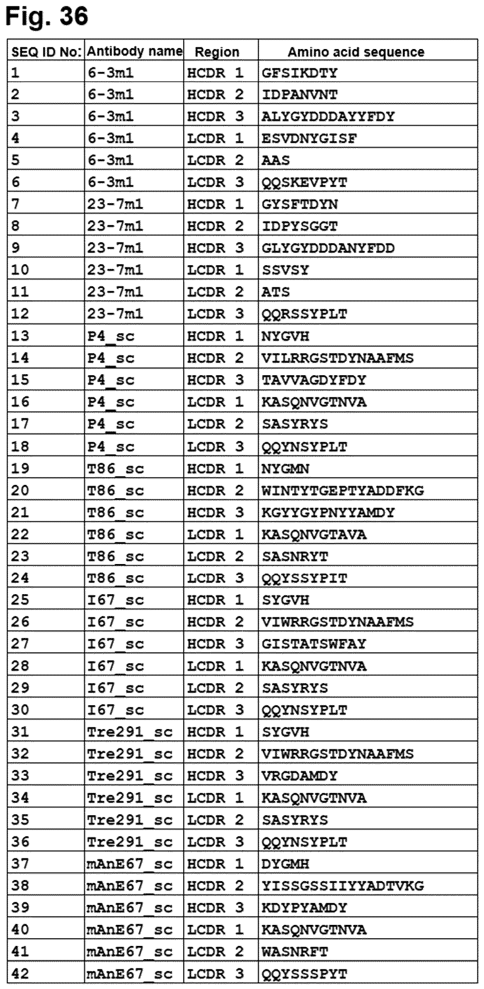

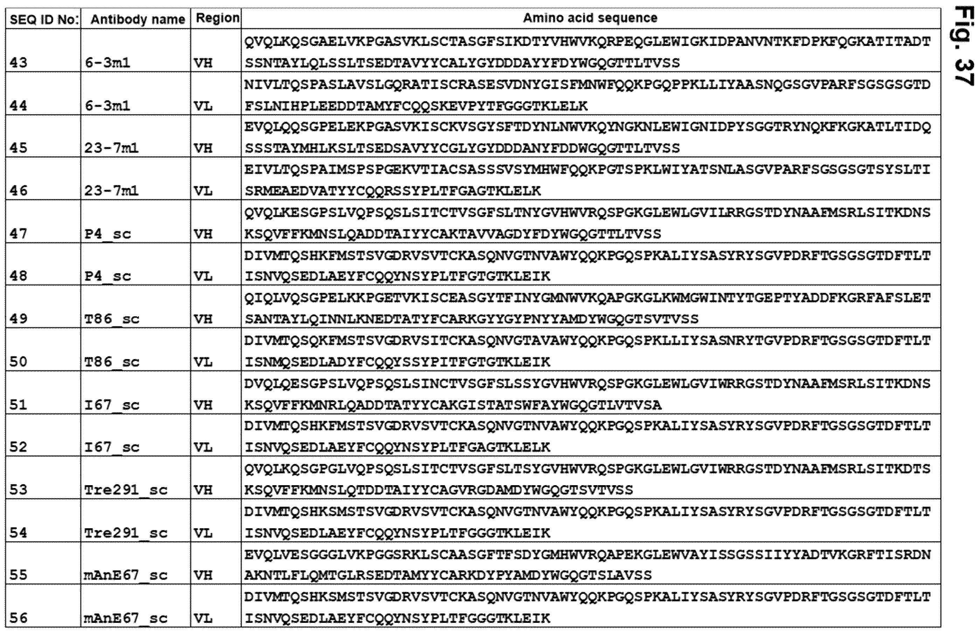

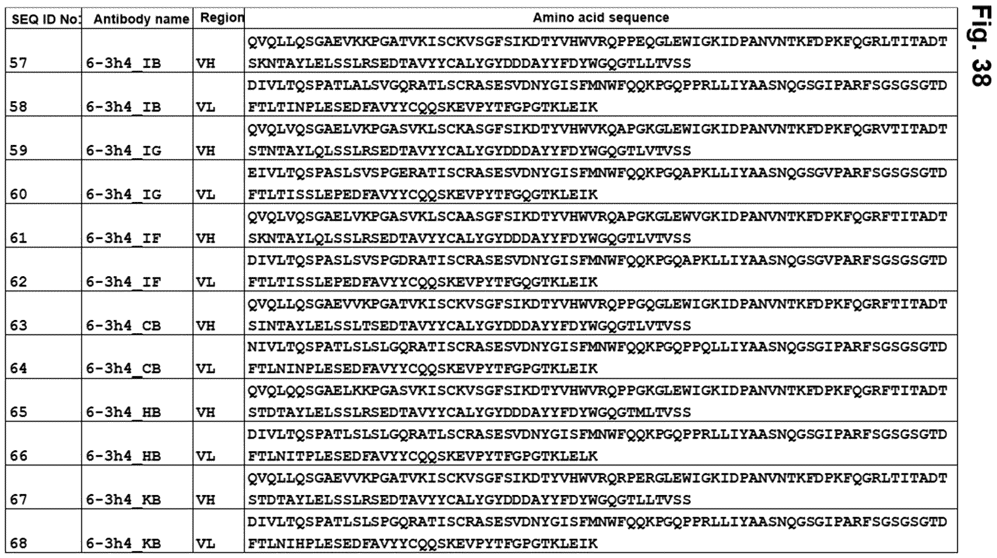

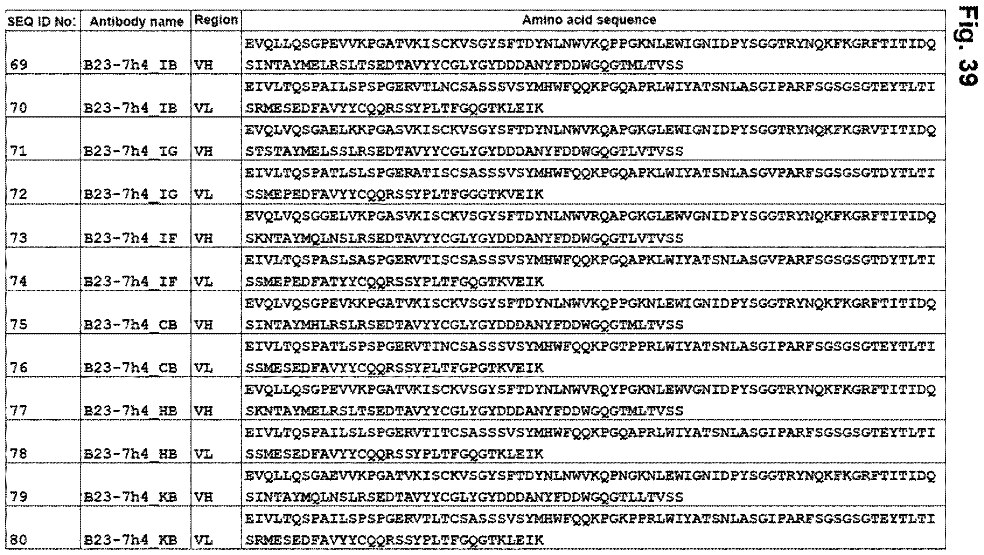

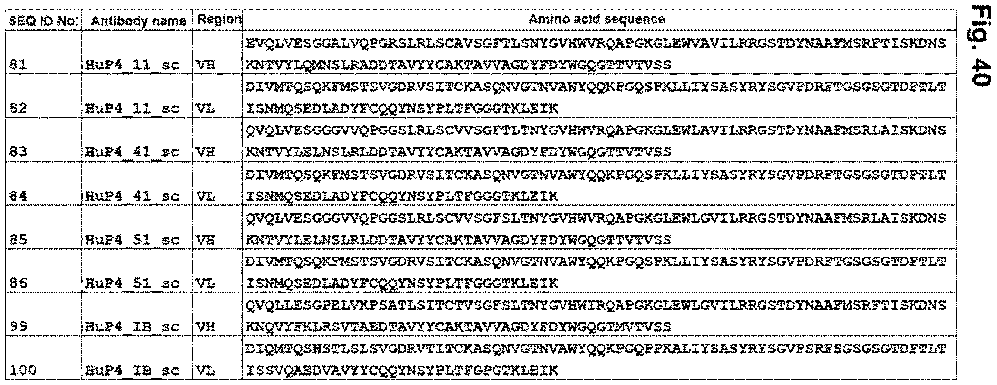

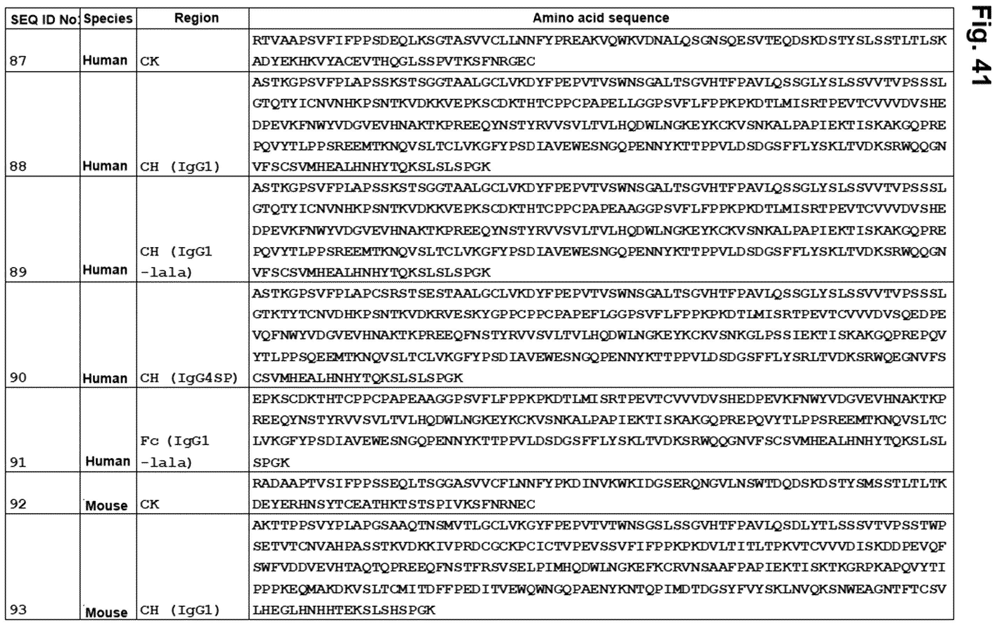

Humanized antibodies obtained by humanizing 6-3m1 were 6-3h4_IB, 6-3h4_IG, 6-3h4_IF, 6-3h4_CB, 6-3h4_HB, and 6-3h4_KB, and humanized antibodies obtained by humanizing 23-7m1 were B23-7h4_IB, B23-7h4_IG, B23-7h4_IF, B23-7h4_CB, B23-7h4_HB, and B23-7h4_KB. These humanized antibodies exhibited favorable CD39 inhibitory activity. Figs. 3, 4A, and 4B show the results of measuring the activity of each antibody to inhibit CD39. In addition, HuP4_11_sc, HuP4_41_sc, HuP4_51_sc, and HuP4_IB_sc were humanized antibodies obtained by humanizing P4_sc. These humanized antibodies exhibited favorable CD39 inhibitory activity. Fig. 5 shows the results of measuring the activity of each antibody to inhibit CD39. The amino acid sequences of the CDRs and variable regions of each antibody obtained in the above experiments are shown in Figs. 36-40 (CDRs of 6-3m1 and 23-7m1 are based on the IMGT definition; CDRs of P4_sc, T86_sc, I67_sc, Tre291_sc, and mAnE67_sc are based on Kabat numbering). The amino acid sequences of the light chain (kappa chain), IgG1-lala heavy chain, and IgG4 heavy chain constant regions used for antibody production are shown in Fig. 41.

### 2.1 To measure affinity (Surface Plasmon Resonance (SPR) analysis)

Kinetics of each antibody against their target antigen were measured using Biacore 8K (Cytiva). Each antibody to be measured was immobilized on a sensor chip (CM5) using a Mouse Antibody Capture Kit, type 2 (Cytiva, 29215281) for mouse antibodies and a Human Antibody Capture Kit, Type 2 (Cytiva, 26234600) for chimeric and humanized antibodies, respectively. Human CD39 antigen was subjected to 4-step dilution from 5×10⁻⁹ M to prepare each solution, the contact time was set to 120 seconds, the dissociation time was set to 600 seconds, and the flow rate was set to 30 µl/min. Under these conditions, HBS-EP+ was used as the running buffer, and each sample was then analyzed using the 1: 1 binding model of multi-cycle kinetics.

### 2.2 Results

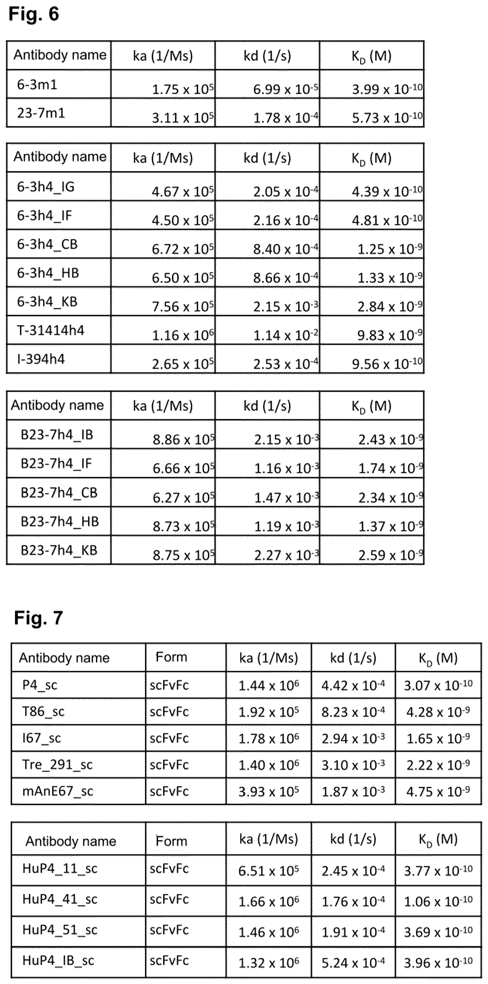

Figs. 6 and 7 show the results of SPR analysis. Here, 6-3m1, 23-7m1, P4_sc, and their humanized antibodies exhibited favorable affinity for human CD39.

### 3.1 CD39 binding analysis (FCM analysis) (using human CD39)

Binding to target antigen-expressing cells was analyzed by FCM analysis using human CD39-overexpressing cells. The human CD39-overexpressing cells were generated using a lentiviral vector system (OriGene, PS100069). Lentiviral particles carrying the human CD39 gene (Gene ID: 953) as the target gene together with the puromycin resistance gene were generated and used to infect HEK293T or Jurkat cells therewith. Then, the cells were cultured in the presence of puromycin. HEK293T or Jurkat cells (239T-hCD39 or Jurkat-hCD39) in which the target gene was integrated into the genome and which became positive for human CD39 were collected and used for FCM analysis. Test or isotype control antibodies at a final concentration of 2 µg/mL or at the concentrations indicated in the figure were reacted with 1 × 10⁵ cells for 15 minutes at 4°C, followed by washing twice with 0.5% BSA, 2 mM EDTA, and D-PBS. After washing, a fluorescent-labeled secondary antibody (anti-human IgG Fc, HP6017) was added, and the cells were stained at 4°C for 15 minutes under light-shielded conditions, followed by washing twice in a similar manner. After addition of 7AAD (BioLegend, 420404), fluorescence intensity was measured using a flow cytometer (FACSVerse, BD). Antibody binding was evaluated based on the fluorescence intensity obtained.

### 3.2 Results

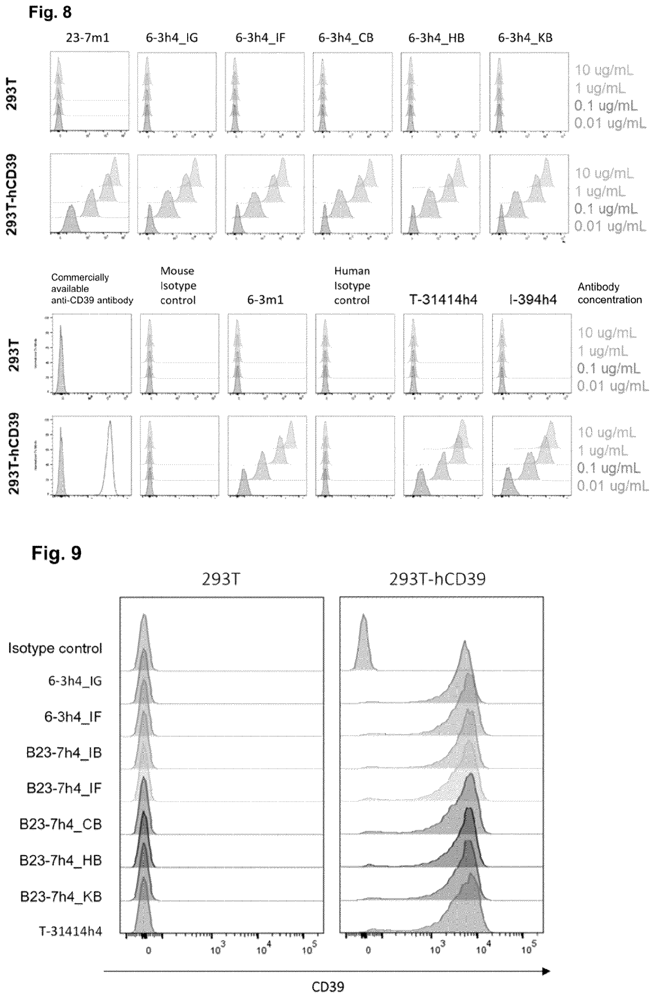

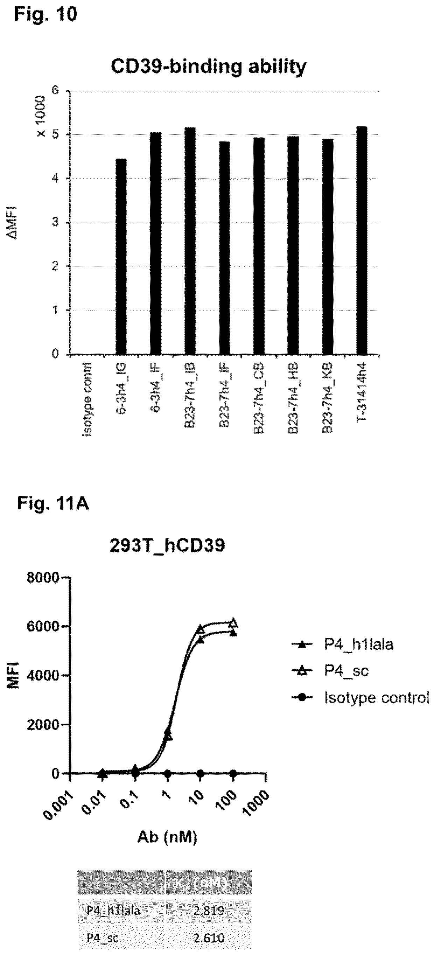

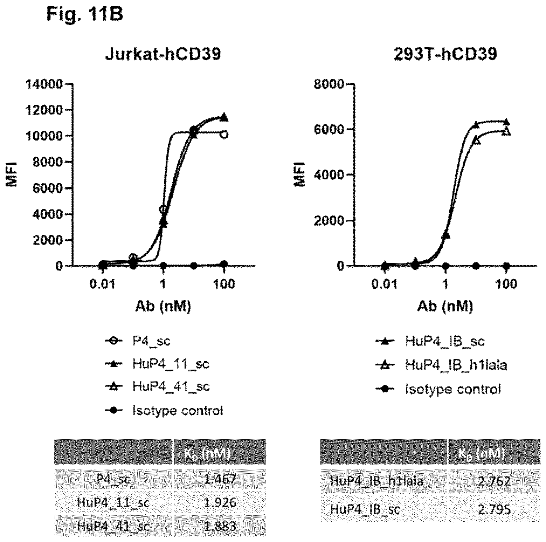

Figs. 8-11B show the results of the FCM analysis. The 6-3m1, 23-7m1, P4_sc, P4_h1lala, and their humanized antibodies exhibited favorable affinity for human CD39 on the cell surface.

### 4.1 CD39 binding analysis (FCM analysis) (using monkey CD39)

Binding to target antigen-expressing cells was analyzed using Cynomolgus monkey CD39-overexpressing cells. This analysis was used to check cross-reactivity. The Cynomolgus monkey CD39-overexpressing cells were generated using a lentiviral vector system (OriGene, PS100069). Lentiviral particles carrying the Cynomolgus monkey CD39 gene (Gene ID: 102132860) as the target gene together with the puromycin resistance gene were generated and used to infect HEK293T cells therewith. Then, the cells were cultured in the presence of puromycin. HEK293T cells (293T-cynoCD39) in which the target gene was integrated into the genome and which became positive for Cynomolgus monkey CD39 were collected and used for FCM analysis. The cells were reacted with 2 µg/ml test antibody or isotype control antibody, stained with a fluorescence-labeled secondary antibody, and evaluated for antibody binding based on the fluorescence intensity obtained by flow cytometry analysis.

### 4.2 Results

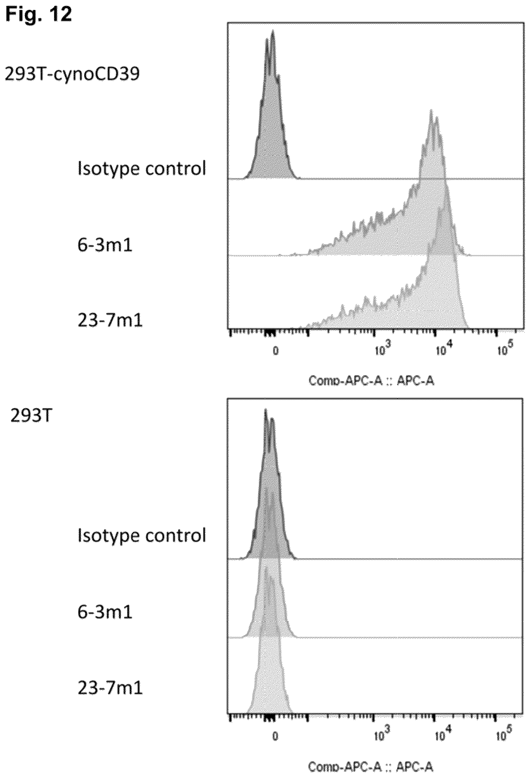

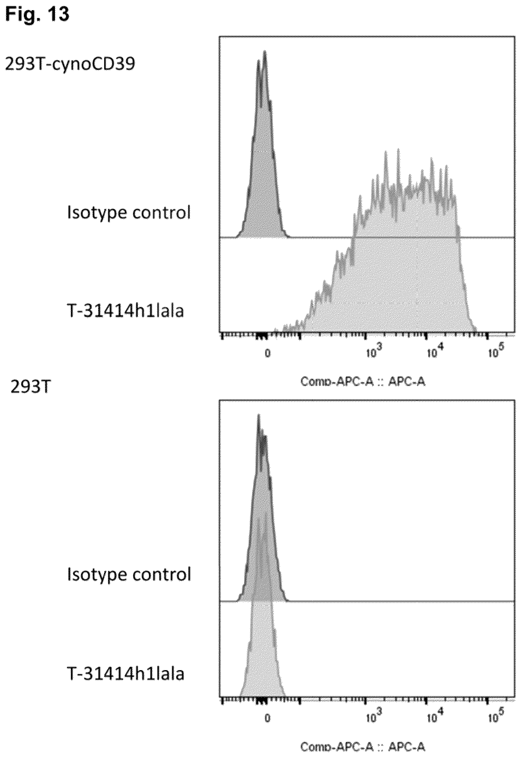

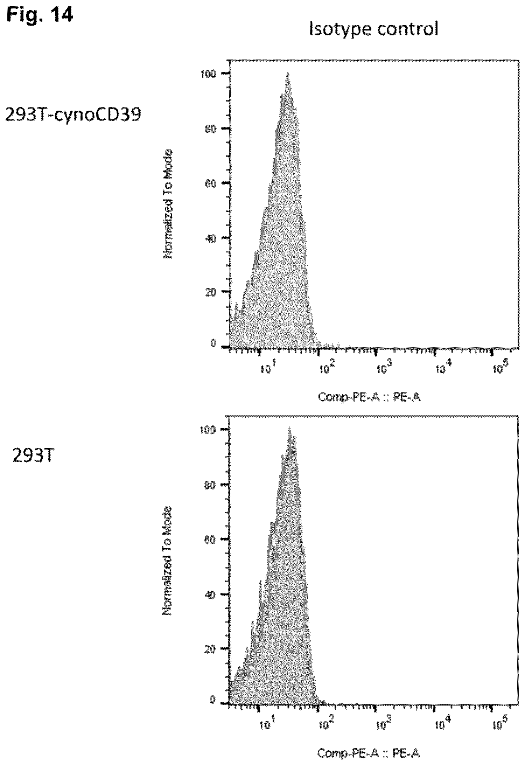

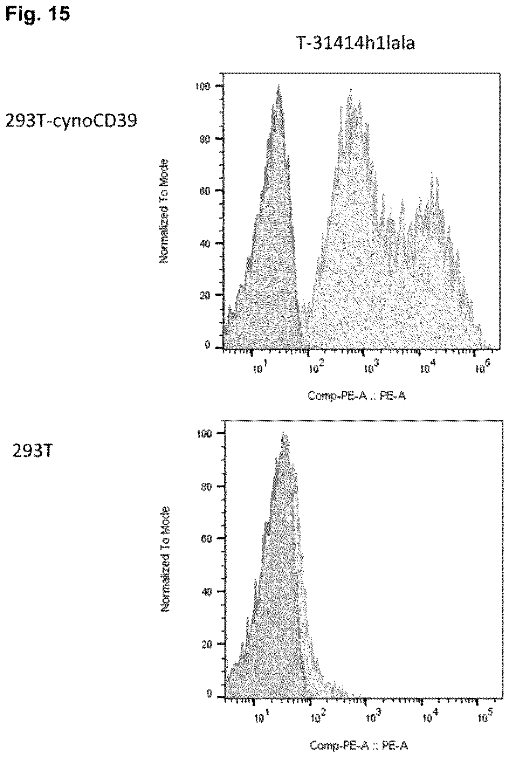

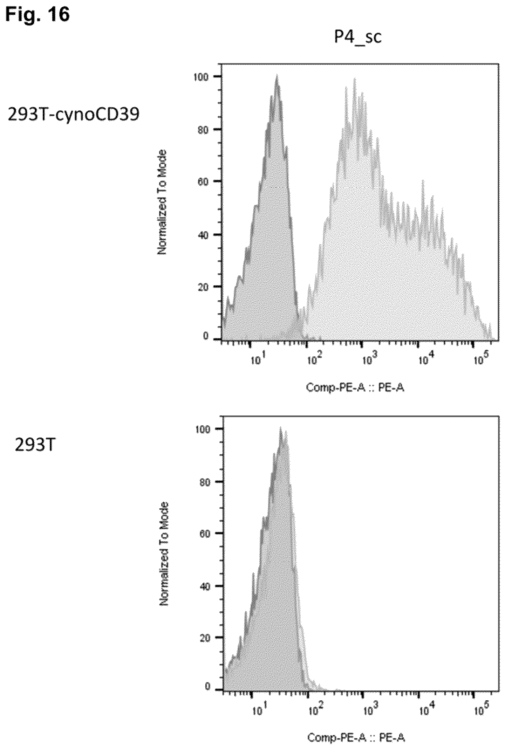

Figs. 12-16 show the results of the FCM analysis. Here, 6-3m1, 23-7m1, and P4_sc also bound to monkey CD39.

### 5.1 Inhibition of and binding to CD39 of Tregs

Purchased healthy human PBMCs (Wako Pure Chemicals, 33000-10M) were seeded at 2 × 10⁵ cells/well in a 96 well U-bottom plate and cultured in the presence of 10 IU/mL IL-2 (NIPRO, 87-890) and 20 ng/mL IL-7 (PeproTech, 200-07) for 6 days. After 6 days, all the cells were collected and the Treg population was isolated using a CD4⁺CD25⁺CD127^{dim/-}Regulatory T Cell Isolation Kit II, human (Miltenyi, 130-094-775). Isolated Tregs were seeded at 2 × 10⁴ cells/well in ab96 well U-bottom plate and stimulated for 6 days in the presence of 6 × 10⁵ Dynabeads Human T-Activator CD3/CD28 (Gibco, DB11132-D), 10 IU/mL IL-2, and 20 ng/mL IL-7. The medium used was RPMI (Wako Pure Chemicals, 189-02025) supplemented with 10 % Human Serum AB (Gemini Bio, 100-512, H46X00K), 1 mM penicillin streptomycin (Nacalai Tesque, 09367-34), 1 mM sodium pyruvate (Gibco, 11360070), 10 mM HEPES (Nacalai Tesque, 17557-94), GlutaMAX (Thermo Fisher, 35050061). 11360070), 10 mM HEPES (Nacalai Tesque, 17557-94), and GlutaMAX (Thermo Fisher, 35050061). Six days later, the Tregs were collected, and analyzed for antibody binding and measured for enzyme inhibitory activity.

The activity of each test antibody to inhibit CD39 enzyme of Tregs was measured using 1 × 10⁴ Treg cells and the test antibody at a final concentration of 10 µg/mL. Like POM1, ARL67156 is a low-molecular-weight inhibitor of E-NTPDases and was used as a positive control for human CD39 inhibition in this experiment.

Binding to Tregs was analyzed by staining the collected cells with Treg surface antigens (anti-hCD127: 236A/E7, anti-hCD3: RPA-T8, anti-hCD25: BC96, anti-hCD4: RPA-T4) and the test antibody at a final concentration of 10 µg/mL, followed by flow cytometry (FACSVerse, BD).

### 5.2 Results

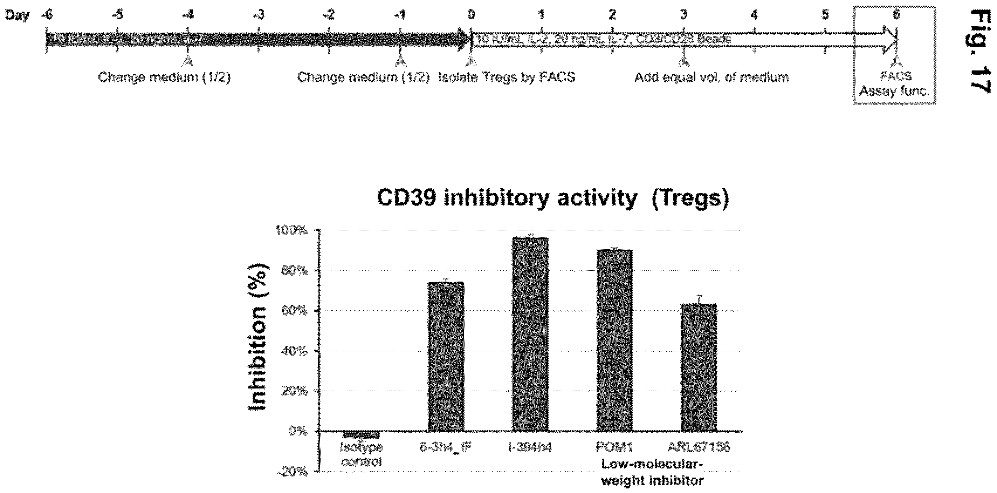

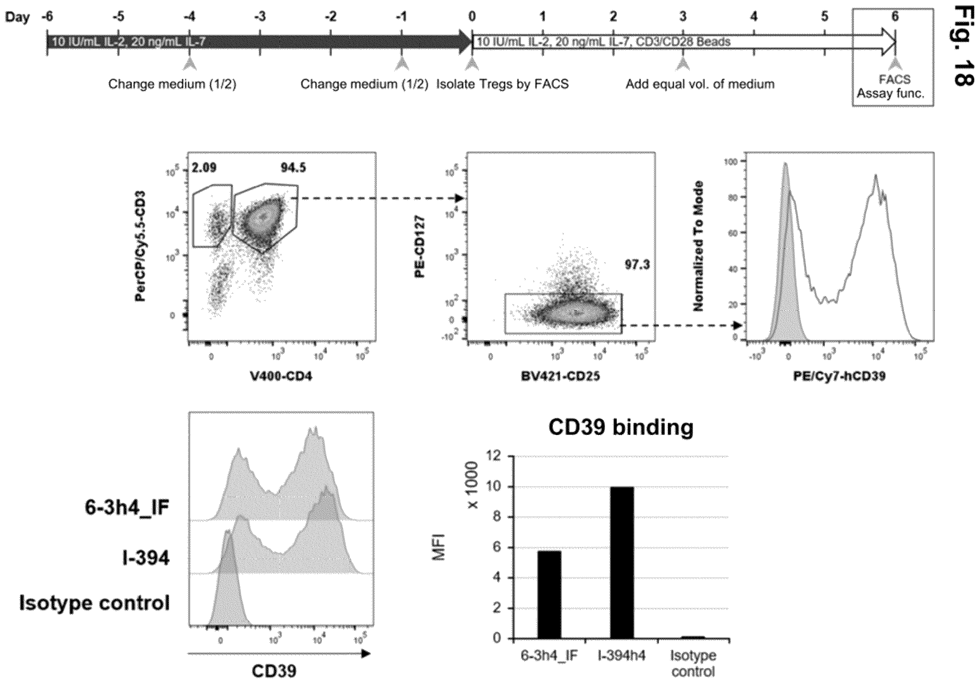

Fig. 17 shows the results of measuring the activity of each antibody to inhibit CD39 enzyme. Here, 6-3h4_IF suppressed the function of CD39 on the surface of Treg cells. Fig. 18 shows the results of CD39 binding analysis. Binding of 6-3h4_IF to Tregs expressing CD39 was found.

### 6.1 T-cell proliferation

Peripheral blood mononuclear cells (PBMCs) from healthy volunteers as suspended in X-VIVO^{™} 15 medium (Lonza, 04-418Q) were seeded (8 × 10⁴ cells/well) onto a 96-well U-bottom plate, and each anti-human CD39 antibody at a final concentration of 0.001 to 10 µg/mL or the corresponding isotype control antibody was added. After incubation for 30 minutes under conditions at 37°C and 5% CO₂, 2 µl of Dynabeads^{™} Human T-Activator CD3/CD28 (VERITAS, DB11132) and 500 µM (final concentration) ATP (Sigma, A2383-1G) were added. The mixture was then cultured under conditions at 37°C and 5% CO₂ for 96 hours. After completion of culture, the cells were stained with T-cell marker staining antibodies (BioLegend; anti-CD3:UCHT1, anti-CD4:RPA-T4, and anti-CD8a:HIT8a). Cell-counting beads were added to a suspension of the stained cells and analyzed by flow cytometry to calculate the number of CD4- or CD8-positive cells per well.

The concentration of TNFα in the collected culture supernatant was measured by the Luminex method using antibodies [capture antibody TNFα: MAB602 (R&D Systems), detection antibody TNFα: BAF210 (R&D Systems)].

### 6.2 Results

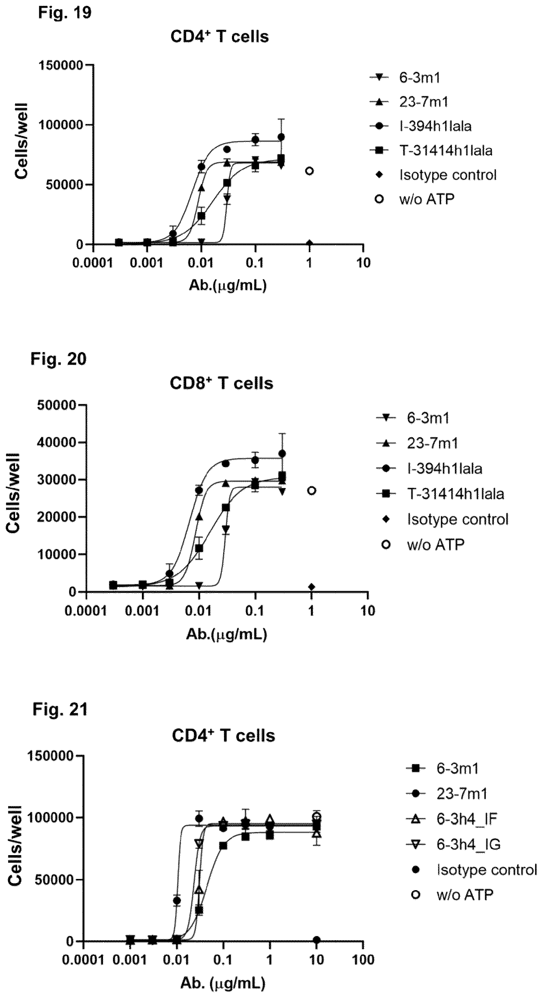

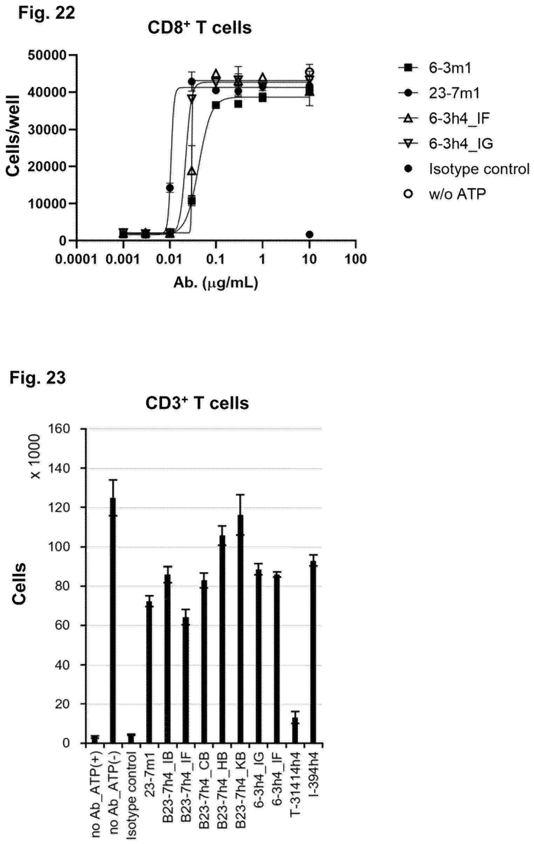

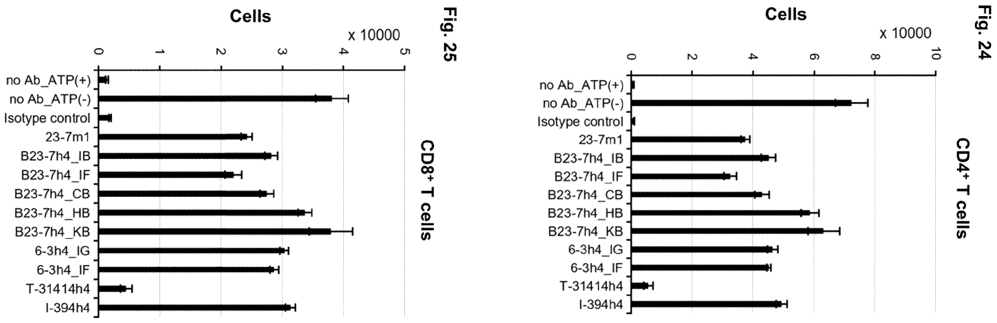

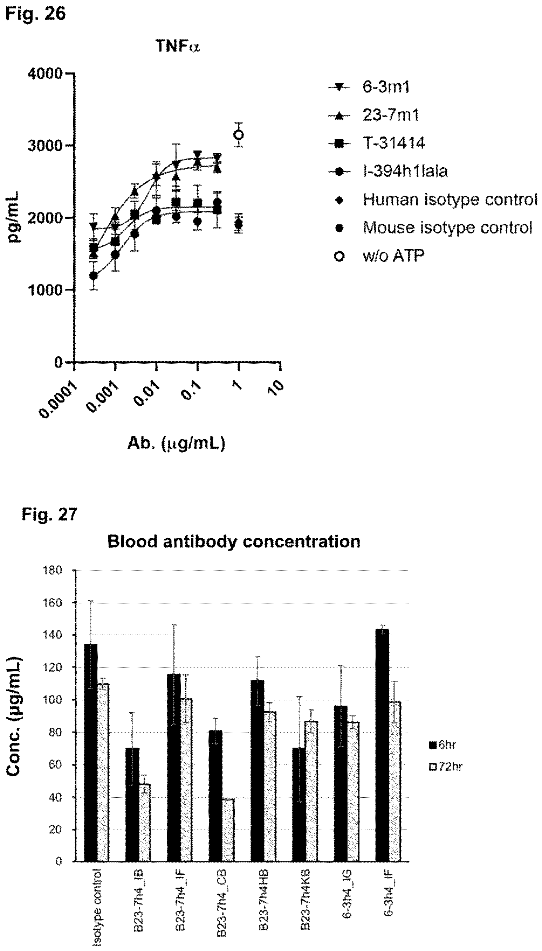

Figs. 19-25 show the results of measuring T-cell proliferation. Here, 6-3m1, 23-7m1, and their humanized antibodies promoted proliferation of CD4- or CD8-positive T cells. Fig. 26 shows the results of measuring the concentration of TNFα. The production of TNFα was significantly enhanced during the 6-3m1 or 23-7m1 treatment when compared to the T-31414h1lala or I-394h4 treatment.

### 7.1 Blood Exposure

SCID mice (C. B-17/Icr-scid/scidJc, female, 6-week-old) were grouped according to their body weight and intraperitoneally received each antibody listed in Table 1. Heparin blood samples were taken 6 and 72 hours after the antibody administration. After centrifugation of peripheral blood at 10,000 × g and at 4°C for 10 min, plasma components were collected and the amount of antibody in the plasma was quantified by ELISA.

**[Table 1]**

| Group number | Administered antibody | Dose |
|---|---|---|
| 1 | Isotype control | 10 mg/kg |
| 2 | B23-7h4_IB | 10 mg/kg |
| 3 | B23-7h4_IF | 10 mg/kg |
| 4 | B23-7h4_CB | 10 mg/kg |
| 5 | B23-7h4_HB | 10 mg/kg |
| 6 | B23-7h4_KB | 10 mg/kg |
| 7 | 6-3h4_IG | 10 mg/kg |
| 8 | 6-3h4_IF | 10 mg/kg |

ELISA was performed according to the following procedure. Anti-IgG (H+L chain) (Human) pAb (MBL, 103G) was immobilized on an EIA/RIA plate (Corning, 9018) at 4°C, overnight, and the plate was washed 3 times with 0.05% Tween-20-containing PBS. Blocking One (Nacalai Tesque, 03953-9) diluted 5-fold with milliQ was added, and blocking was performed for 2.5 hours at room temperature. After washing the plate three times, diluted plasma was added and reacted for 1 hour at room temperature. After washing the plate three times, 1/8000 diluted anti-IgG (γ chain) (Human) pAb-HRP was added and reacted for 30 minutes at room temperature. After completion of the reaction, the plate was washed three times and reacted with a TMB One-Step Substr System (Agilent Technologies, S159985-2) for 10 min at room temperature in the dark. TMB and 0.5 mol/L sulfuric acid (FUJIFILM Wako Pure Chemicals, 192-04755) in an equal volume were added, the reaction was stopped, and absorbance was then measured using a Varioskan LUX (Thermo).

### 7.2 Results

Fig. 27 shows the concentration of antibody after exposed in blood. Each antibody had a favorable concentration after exposure in blood.

### 8.1 Anti-tumor activity

The human lung cancer cell line NCI-H292 having red fluorescent protein (RFP) was seeded on a 96-well plate and cultured overnight. The next day, after removal of the medium, IL-2 (Miltenyi, 130-097-745) at a final concentration of 30 IU as suspended in X-VIVO-15^{™} medium (Lonza, 04-418Q) and an anti-human CD39 antibody or isotype control antibody at a final concentration of 0.3 to 100 µg/mL were added and the mixture was incubated at 37°C for 30 minutes. Then, PBMCs from healthy volunteers stimulated with DynabeadsTM Human T-Activator CD3/CD28 (VERITAS, DB11131) for 10 days were added at an E/T ratio of 2 and the mixture was incubated at 37°C for 30 minutes. After that, ATP was added at a final concentration of 500 or 250 µM, and changes over time in cancer cell growth were observed in the image data by using the IncuCyte ZOOM System (Sartorius) set up at 37°C and 5% CO₂. This made it possible to evaluate the anti-tumor activity of the antibody due to its ability to promote the cytotoxic activity of PBMCs.

### 8.2 Results

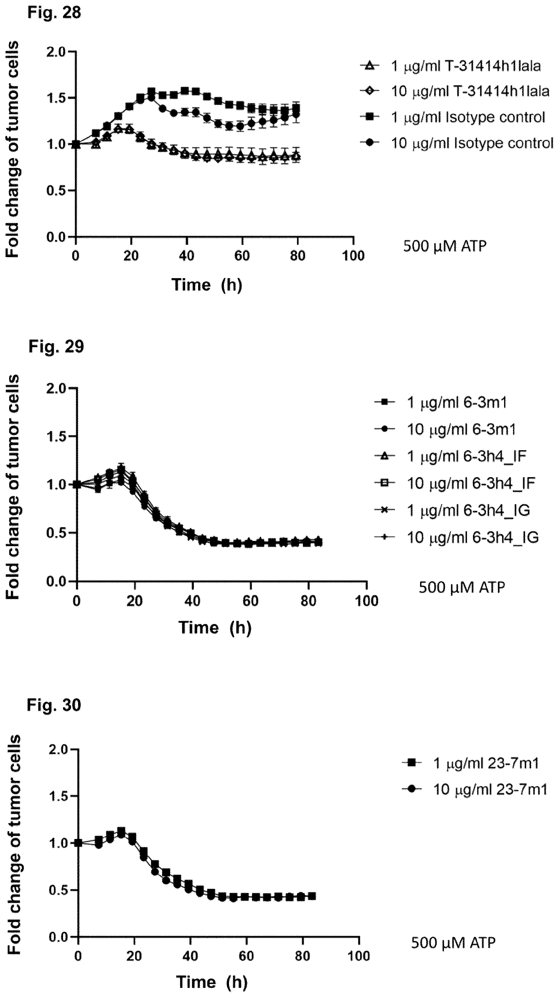

Figs. 28-32 show the results of measuring anti-tumor activity of each antibody. In the case of treatment with the isotype control, the growth rate of cancer cells increased (Fig. 28). ATP (500 µM) added to the experimental system is thought to have caused immunosuppression, leading to an increase in the growth rate of cancer cells. In the case of treatment with T-31414h1lala, the growth rate of cancer cells decreased, but only to a small extent (approximately 0.9) (Fig. 28). On the other hand, in the case of treatment with 6-3m1, its humanized antibody, or 23-7m1, the growth rate of cancer cells markedly decreased (to about 0.4) (Figs. 29 and 30). There was no significant difference in the growth rate of cancer cells among clones of 6-3m1, its humanized antibody, and 23-7m1.

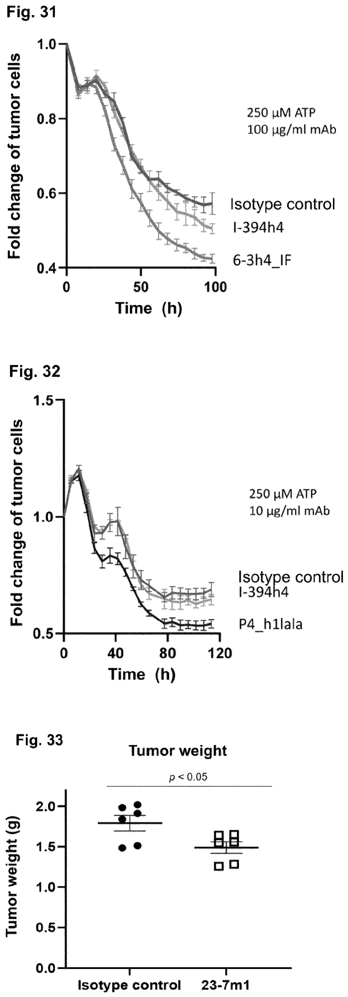

Fig. 31 shows the results of measuring anti-tumor activity of 6-3h4-IF. This measurement was performed under conditions at a reduced ATP amount (250 µM). In the case of treatment with the isotype control, the growth was gradually inhibited up to 100 hours after treatment. The low amount of ATP added to the experimental system is thought to have created an environment in which immunosuppression was less likely to occur, resulting in the gradual suppression of cancer cell growth. In the case of treatment with I-394h4, there was no difference when compared to the isotype control until about 50 hours after treatment. A slight difference was then observed, but the overall difference was small. In the case of treatment with 6-3h4-IF, a difference started to appear when compared to the isotype control at about 15 hours after treatment. The difference continued to increase, and the overall difference was large. Thus, it was shown that 6-3h4-IF exerts cancer cell growth inhibitory effects in a short period of time even in an environment where immunosuppression is unlikely to occur, and that the degree of growth suppression was large.

Fig. 32 shows the results of measuring anti-tumor activity of P4_h1lala. This measurement was performed under conditions at a reduced ATP amount (250 µM) and antibody concentration (10 µg/ml). In the case of treatment with the isotype control, the growth was gradually inhibited up to 80 hours after treatment. The low amount of ATP added to the experimental system is thought to have created an environment in which immunosuppression was less likely to occur, resulting in the gradual suppression of cancer cell growth. In the case of treatment with I-394h4, there was no difference when compared to the isotype control until about 80 hours after treatment. Thereafter, only a slight difference was observed. In the case of treatment with P4_h1lala, a difference started to appear when compared to the isotype control at about 20 hours after treatment. The difference continued to increase, and the overall difference was large. Thus, it was shown that P4_h1lala exerted cancer cell growth inhibitory effects in a short period of time even in an environment where immunosuppression is unlikely to occur, that the degree of growth suppression was large.

### 9.1 Anti-tumor activity (in vivo, 23-7m1 single drug)

The human multiple myeloma-derived cell line MOLP-8 (DMSZ, ACC569) was subcutaneously transplanted (at 5 × 10⁶ cells/mouse) into the right abdomen of each SCID mouse (C.B-17/Icr-scid/scidJcl, female, 7-week-old). On day 11 after transplantation, the mice were divided into groups based on their tumor volume, and each antibody listed in Table 2 was administered twice a week for a total of 6 doses. On day 34 after transplantation, mice were euthanized and the tumors were collected and weighed. The difference in tumor weight was analyzed by Student's t-test. The significance level was set to 5%.

**[Table 2]**

| Group number | Administered antibody | Dose |
|---|---|---|
| 1 | Mouse IgG1 isotype control | 10 mg/kg |
| 2 | 23-7m1 (Mouse IgG1) | 10 mg/kg |

### 9.2 Results

Fig. 33 shows the results of measuring *in vivo* anti-tumor activity. The tumor weight was significantly reduced in the 23-7m1 administration group compared to the isotype control antibody administration group. Thus, 23-7m1 has been demonstrated to have anti-tumor activity *in vivo.*

### 10.1 Anti-tumor activity (in vivo, 23-7m1 single drug)

The human multiple myeloma-derived cell line MOLP-8 (DMSZ, ACC569) was subcutaneously transplanted (at 5 × 10⁶ cells/mouse) into the right abdomen of each SCID mouse (C.B-17/Icr-scid/scidJcl, female, 7-week-old). On day 10 after transplantation, the mice were divided into groups based on tumor volume. Each antibody was administered in the doses listed in Table 3 twice a week for a total of seven doses starting on the day of grouping. Doxorubicin was administered twice a week for a total of 5 doses, starting 7 days after the grouping date. The tumor diameter and body weight were measured on day 24 after grouping. The tumor weight (n=5) data were analyzed using GraphPad Prism 9.2.0. One-way ANOVA and Tukey multiple comparisons test were used for statistical analysis. A significant difference was assumed when p** ≤ 0.01.

**[Table 3]**

| Group | Administered antibody | Dose |
|---|---|---|
| 1 | Isotype control | 10 mg/kg |
| | Solvent | 1.5 mg/kg |
| 2 | Isotype control | 10 mg/kg |
| | Doxorubicin | 1.5 mg/kg |
| 3 | 23-7m1 | 10 mg/kg |
| | Doxorubicin | 1.5 mg/kg |

### 10.2 Results

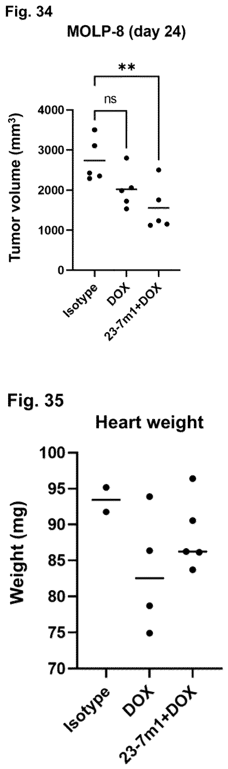

Fig. 34 shows the results of measuring the *in vivo* anti-tumor activity (of 23-7m1 and DOX used in combination) (on day 24). The tumor volume was significantly reduced in the 23-7m1 and doxorubicin combination group compared to the isotype control or solvent control group. Fig. 35 shows the results of measuring the heart weight (on day 24). The heart weight was decreased on the last day of the study in the group of treatment with doxorubicin alone. This decrease tended to be mitigated in the 23-7m1 and doxorubicin combination group.

### 11.1 To evaluate Treg Function

Teff (effector T cells) (1 × 10⁴ cells) isolated from CytoTell Green (AAT Bioquest)-labeled human PBMCs and Tregs isolated from human PBMCs are seeded in a 96-well plate at a ratio of 1:4 to 32. Dynabeads^{™} Human T-Activator CD3/CD28 (VERITAS) are added and the mixture is cultured in serum-free X-VIVO 15 medium with ATP at a final concentration of 500 µM. After 3-5 days, the fluorescence intensity of CytoTell Green is measured using a flow cytometer to determine Teff division. An anti-human CD39 antibody or isotype control antibody at a final concentration of 0.3 to 100 µg/mL is used as the test drug. The anti-human CD39 antibody releases Treg-mediated inhibition of Teff division.

### 12.1 To evaluate Treg removal

12.1.1 Human PBMCs and Tregs isolated from human PBMCs are co-cultured at an effector:target cell (E:T) ratio of 40:1, 10:1, 5:1, and 1:2. Six hours later, TO-PRO-3 iodide (642/661; Invitrogen/Molecular Probes) is added, and cell death is measured by flow cytometry. An anti-human CD39 antibody or isotype control antibody at a final concentration of 0.3 to 100 µg/mL is used as the test drug. The anti-human CD39 antibody lyses Tregs via ADCC.

12.1.2 Purchased healthy human PBMCs (Wako Pure Chemicals, 33000-10M) are seeded at 2 × 10⁵ cells/well in a 96 well U-bottom plate and cultured in the presence of 20 IU/mL IL-2 (NIPRO, 87-890) and 40 ng/mL IL-7 (PeproTech, 200-07) for 6 days. After culture, the frequency of Tregs is analyzed by flow cytometry. An anti-human CD39 antibody or isotype control antibody at a final concentration of 0.3 to 100 µg/mL is used as the test drug. The anti-human CD39 antibody decreases the frequency of Tregs.

### 13.1 To prepare glycan-modified antibodies 6-3h1_IF_NF1 to 3

First, an expression vector was created by recombining the heavy-chain constant region sequence of the expression vector containing 6-3h4_IF from human CH (IgG4SP) to human CH (IgG1), thereby changing the subclass from IgG4 to IgG1. The antibody expressed from this vector was named 6-3h1_IF (the VH and VL amino acid sequences are the same as those of 6-3h4_IF; the amino acid sequence of human CH (IgG1) is represented by SEQ ID NO: 88). The glycan-modified antibodies obtained in the later experiments were named 6-3h1_IF_NF1, 6-3h1_IF_NF2, and 6-3h1_IF_NF3. The above expression vector was then transfected into CHO cells using an ExpiCHO Expression System Kit (Thermo Fisher Scientific, A29133) to generate transiently antibody-expressing CHO cells. On the day before transfection, a final concentration of 20 mM D-(-)-arabinose (Nacalai Tesque, 03330-32) was added to ExpiCHO Expression Medium in each culture flask for 6-3h1_IF_NF1. For 6-3h1_IF_NF2, a final concentration of 20 µM 2F-Peracetyl-Fucose (DMSO suspension) (Merck, 344827) was added to ExpiCHO Expression Medium. For 6-3h1_IF_NF3, a final concentration of 20 mM D-(-)-arabinose and a final concentration of 20 µM 2F-Peracetyl-Fucose were added to ExpiCHO Expression Medium. After transfection, the cells were cultured according to the ExpiCHO Expression System Kit's instructions. For 6-3h1_IF _NF2 and NF3, 2F-Peracetyl-Fucose at a final concentration of 20 µM was added to the culture flask with shaking on day 6, when day 0 was set to the day of transfection, and the culture was continued. For each antibody, the antibody produced in the culture supernatant from CHO cells was collected and purified on day 7 of culture.

### 13.2 To measure affinity (SPR analysis)

Substantially the same method as in the Section 2.1 above was used to measure the affinity of 6-3h1_IF_NF1 to 3 for human CD39.

### 13.3 Results

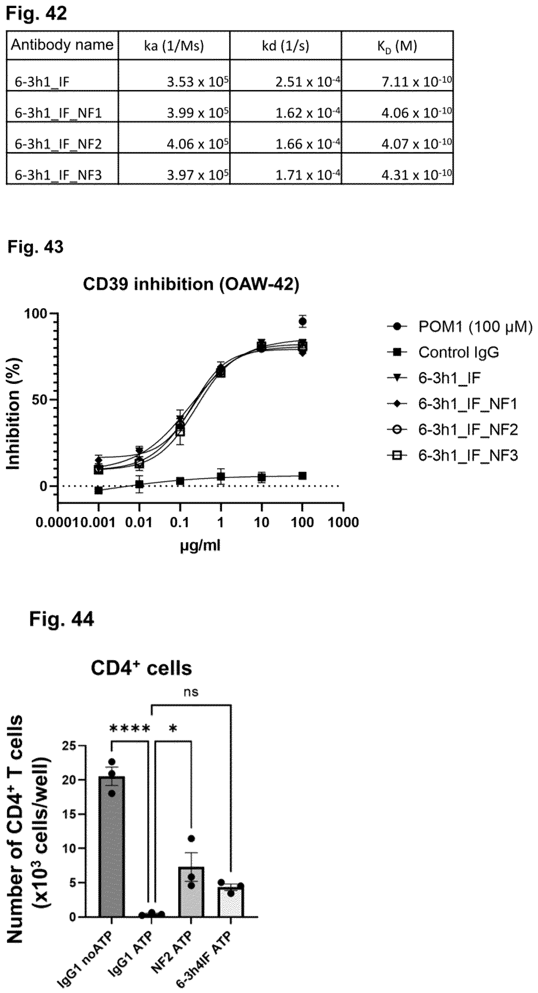

Fig. 42 shows the results of SPR analysis. The affinity for human CD39 was almost the same K_{D} value regardless of the presence or absence of glycan modification or the glycan modification method.

### 14.1 To measure activity to inhibit CD39 enzyme

Substantially the same method as in the Section 1.8 above was used to measure the activity of 6-3h1_IF_NF1 to 3 to inhibit human CD39.

### 14.2 Results

Fig. 43 shows the results of measuring the enzyme inhibitory activity. The human CD39 inhibitory activity was almost the same value regardless of the presence or absence of glycan modification or the glycan modification method.

### 15.1 To test T-cell proliferation mediated by suppressing CD39 function

Frozen PBMCs (Wako, 551-37651, Lot: 3010116139) were thawed and pan T cells were isolated (using a Pan T Cell Isolation Kit, human, Miltenyi Biotec, 130-096-535). Cells stained with 0.2 µl CytoTell^{™} UltraGreen (AAT Bioquest) were suspended in X-VIVOTM 15 Serum-free Hematopoietic Cell Medium (Lonza, BE02-060F) and seeded at 8 × 10⁴ cells/25 µl in a 96-well U-bottom plate (mitomycin C treatment (10 µg/mL) (Nacalai Tesque, 20888-21) was used as a non-division control). Next, 25 µl of 4× test antibody (40 µg/mL) was each added and incubated at 37°C and 5% CO₂ for 30 min, followed by 25 µl of 4× ATP (1400 µM) (SIGMA, A2383-1G) and 25 µl of 4× CD3 (40 µg/mL)/CD28 (8 µg/mL) mixture. The cells were then incubated at 37°C and 5% CO₂ for 96 hours. Surface antigens were stained with various antibodies (PerCP/Cy5.5-CD8, APC-CD3, APC/Cy7-Zombie-NIR, V500-CD4). Absolute Counting Beads (Invitrogen, C36950) were added, followed by analysis using a FACS Verse. GraphPad Prism 9.2.0 was used for data analysis. One-way ANOVA and Dunnet method were used for statistical analysis. p*≤0.05, p**≤0.01, p***≤0.001, or p****≤0.0001 was considered significantly different.

### 15.2 Results

Fig. 44 shows the results of measuring the number of CD4⁺ T cells. CD4⁺ T cell counts were significantly and markedly reduced by the addition of ATP (see IgG1 ATP; IgG1 is the control antibody). Addition of 6-3h1_IF_NF2 significantly mitigated this decrease (see NF2 ATP). The IgG4 type 6-3h4IF showed a mitigation tendency when compared to 6-3h1_IF_NF2, but not significantly (see 6-3h4IF ATP).

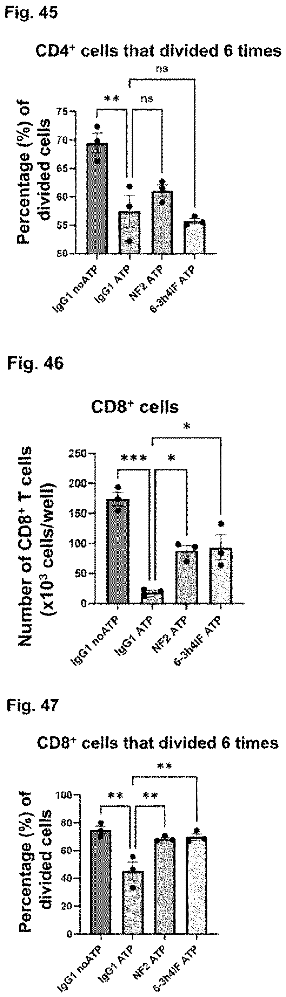

Fig. 45 shows the percentage of CD4⁺ T cells that divided six times. CD4⁺ T cell division was significantly reduced by the addition of ATP. Addition of 6-3h1_IF_NF2 or 6-3h4IF did not show significant improvement.

Fig. 46 shows the results of measuring the number of CD8⁺ T cells. CD8⁺ T cell count was significantly and markedly reduced by the addition of ATP. Addition of 6-3h1_IF_NF2 or IgG4-type 6-3h4IF significantly mitigated this decrease.

Fig. 47 shows the percentage of CD8⁺ T cells that divided six times. CD8⁺ T cell division was significantly reduced by the addition of ATP. Addition of 6-3h1_IF_NF2 or 6-3h4IF significantly mitigated this decrease.

### 16.1 To analyze glycan of 6-3h1_IF_NF1 to 3

Here, 50 µg of each test antibody was processed according to the instructions of Antibody N-type Glycan Analysis Kit EZGlyco^{™} (Sumitomo Bakelite, BS-X4410) to prepare 2-aminobenzamide-labeled N-type glycan. The prepared samples were analyzed by Glycan, LC-MS measurement service (for N-type glycan) (Sumitomo Bakelite, BS-X4913). The fucosylation percentage was calculated from the percentage of the peak area where core fucose was presumably added while the estimated glycan structure was analyzed (fucosylation percentage (%) = (peak area of core fucose-containing glycan) / (peak area of the entire N-type glycan) × 100). Human serum IgG served as the experimental control for glycan structure analysis.

### 16.2 Results

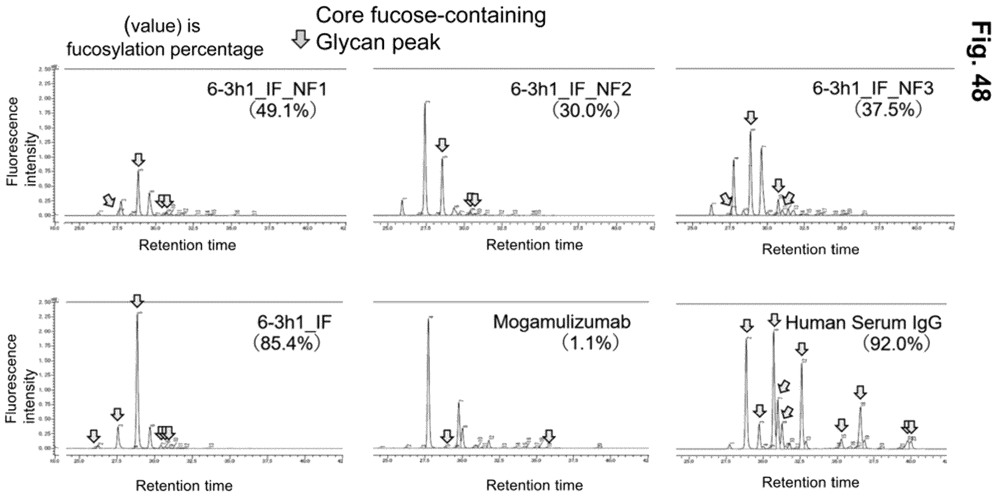

Fig. 48 shows the results of glycan analysis (LC chromatogram). The fucosylation percentages of 6-3h1_IF and 6-3h1_IF_NF1 to 3 were 85.4%, 49.1%, 30.0%, and 37.5%, respectively. Core fucose addition in each glycan-modified antibody was suppressed, with 6-3h1_IF_NF2 being the most suppressed.

### 17.1 To analyze binding to activated Tregs

Purchased healthy human PBMCs (Wako Pure Chemicals, 33000-10M) were seeded at 2 × 10⁵ cells/well in a 96 well U-bottom plate and cultured in the presence of 10 IU/mL IL-2 (NIPRO, 87-890) and 20 ng/mL IL-7 (PeproTech, 200-07) for 6 days. After 6 days, all the cells were collected and the Treg population was isolated using a CD4⁺CD25⁺CD127^{dim/-} Regulatory T Cell Isolation Kit II, human (Miltenyi, 130-094-775). Isolated Tregs were seeded at 2 × 10⁴ cells/well in ab96 well U-bottom plate and stimulated for 6 days in the presence of 6 × 10⁵ Dynabeads Human T-Activator CD3/CD28 (Gibco, DB11132-D), 10 IU/mL IL-2, and 20 ng/mL IL-7. The medium used was RPMI (Wako Pure Chemicals, 189-02025) supplemented with 10 % Human Serum AB (Gemini Bio, 100-512, H46X00K), 1 mM penicillin streptomycin (Nacalai Tesque, 09367-34), 1 mM sodium pyruvate (Gibco, 11360070), 10 mM HEPES (Nacalai Tesque, 17557-94), GlutaMAX (Thermo Fisher, 35050061). 11360070), 10 mM HEPES (Nacalai Tesque, 17557-94), and GlutaMAX (Thermo Fisher, 35050061). After 6 days, Tregs were collected and analyzed for binding of each test antibody by using a FACS Verse. GraphPad Prism 9.2.0 was used for data analysis.

### 17.2 Results

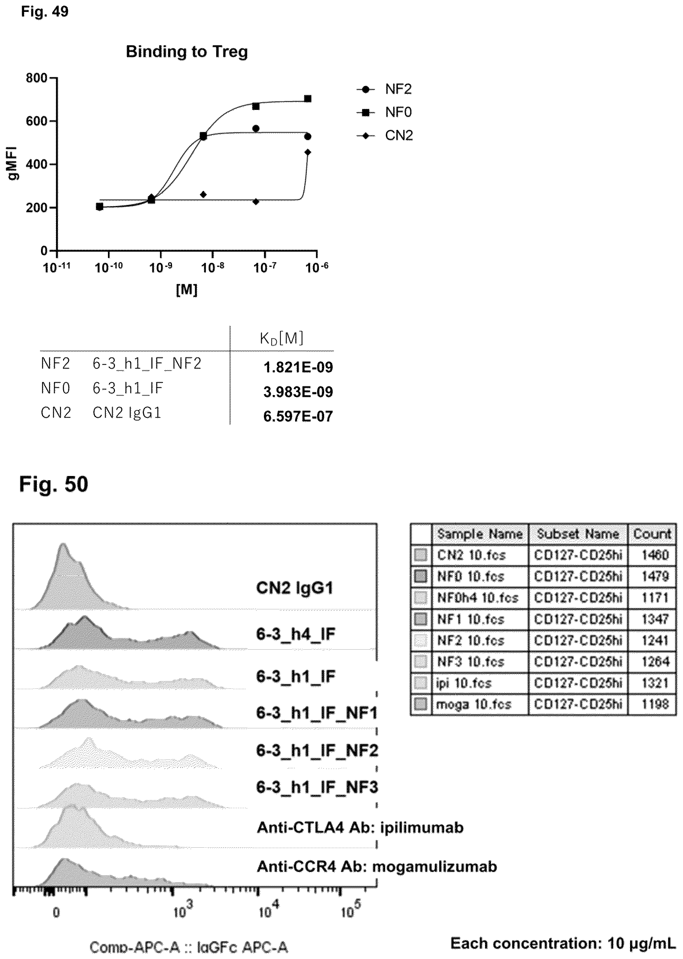

Figs. 49 and 50 show the results of binding analysis. The affinity (K_{D} [M]) of 6-3h1_IF or 6-3h1_IF_NF2 for activated Tregs was 3.983 × 10⁹ or 1.821 × 10⁹, respectively. No difference in the ability of binding to activated Tregs at a concentration of 10 µg/mL was present among IgG4-type 6-3h4IF, IgG1-type 6-3h1_IF, and 6-3h1_IF_NF1 to 3. In comparison, ipilimumab (ipi, YERVOY (registered trademark), Bristol Myers Squibb) and mogamulizumab (moga, POTELIGEO (registered trademark), Kyowa Kirin) showed that the ability of binding to activated Tregs was not strong.

### 18.1 To measure ADCC activity (reporter assay)

Activated Tregs were seeded as target cells at 3 × 10⁴ cells/well and treated with 0.0001 to 10 µg/ml of each test antibody according to the instructions of ADCC Reporter Bioassay, V Variant (Promega, G7010). The ADCC activity value (fold change) was defined as the relative value of ADCC activity when the value at the time of addition of control IgG 1 antibody at each concentration was set as 1.

### 18.2 Results

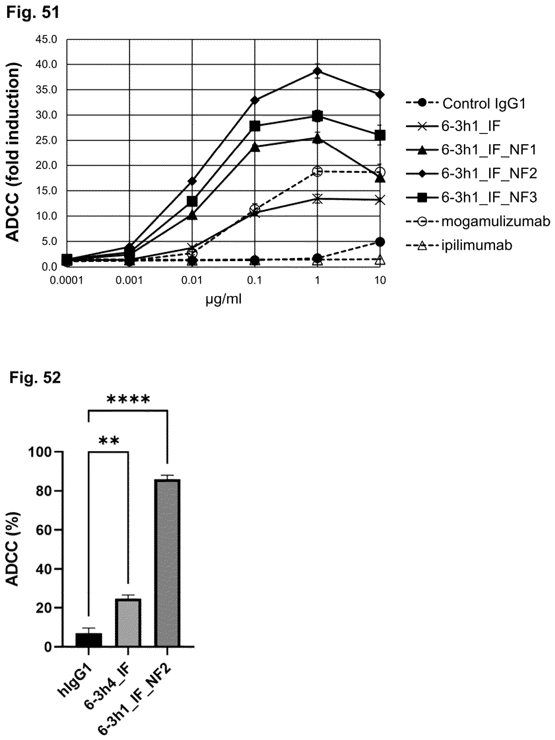

Fig. 51 shows the results of measuring the ADCC activity. The ADCC activity of 6-3h1_IF_NF2 was the highest, followed by NF3 and NF1, all of which exceeded the ADCC activity of 6-3h1_IF before glycan modification.

### 19.1 To measure ADCC activity (against cancer cell line)

The human ovarian cancer cell line OVA42 was seeded in a 96-well plate and cultured overnight. The next day, after the medium was removed, each test antibody or isotype control at a final concentration of 10 µg/mL was added, and PBMCs from healthy volunteers were added at an E/T ratio of 10. After centrifugation at 200 G for 3 minutes, the cells were cultured under conditions at 37°C and 5% CO₂ for 18 hours. After the end of culture, the number of dead cells was counted using a CytoTox-Glo^{™} Cytotoxicity Assay (Promega G9290). The ADCC activity was calculated using RLU values by the following formula: ADCC activity (%) = (RLU value for the antibody solution at each concentration when effector cells and target cells are mixed - RLU value when effector cells and target cells are mixed) / (RLU value when target cells are admixed with Lysis Buffer - RLU value when target cells alone are cultured) × 100. GraphPad Prism 9.2.0 was used for data analysis. One-way ANOVA and Dunnet method were used for statistical analysis. p**≤0.01 or p****≤0.0001 was considered significantly different.

### 19.2 Results

Fig. 52 shows the results of measuring the ADCC activity. Here, 6-3h1_IF_NF2 (10 µg/mL) exhibited significant and marked ADCC activity against human ovarian cancer strain OAW-42.

### 20.1 To measure affinity (SPR analysis) of 6-3h1_IF_NF1-3 for FcγRIIIa

Kinetics of each antibody against their target antigen were measured using Biacore 8K (Cytiva). CD16a Protein, Human, Recombinant (ECD, F176V, His Tag) (Sino Biological, 10389-H08H1) labeled using a Biotinylation Kit (EZ-Link Sulfo-NHS-LC-Biotin) (Thermo Fisher Scientific, A39257) was immobilized on a Series S Sensor Chip CAP (Cytiva, 28920234) by using a Biotin CAPture Kit, Series S (Cytiva, 28-9201-34). Each test antibody was subjected to 7-step dilution from 2×10⁻⁶ M to prepare each solution, the contact time was set to 60 seconds, the dissociation time was set to 600 seconds, and the flow rate was set to 30 µl/min. Under these conditions, HBS-EP+ was used as the running buffer, and each sample was then analyzed using the 1:1 binding model of multi-cycle kinetics.

### 20.2 Results

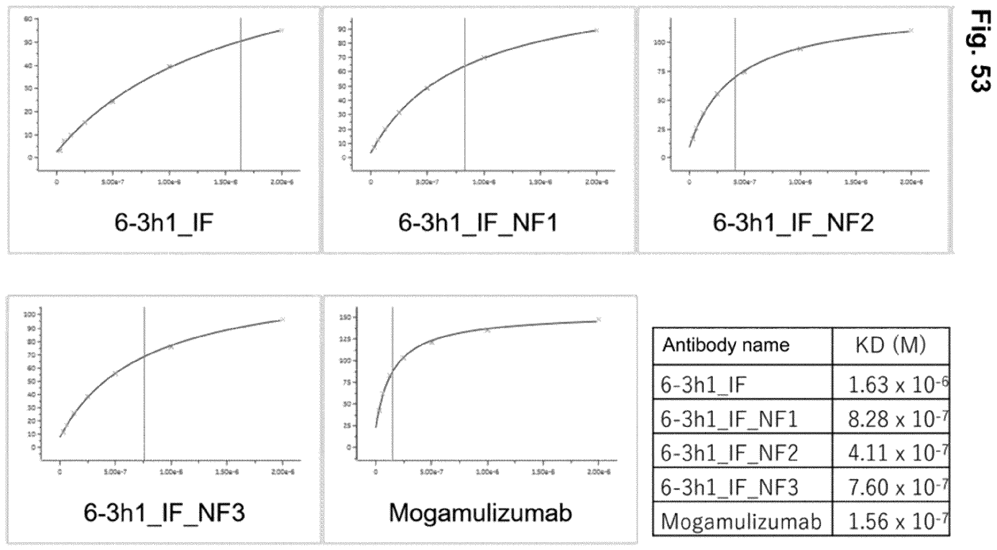

Fig. 53 shows the results of SPR analysis. Any of the glycan-modified antibodies 6-3h1_IF_NF1 to 3 exhibited higher affinity for FcγRIIIa (V variant) than the unmodified antibody 6-3h1_IF, and the affinity of NF2 was the best.

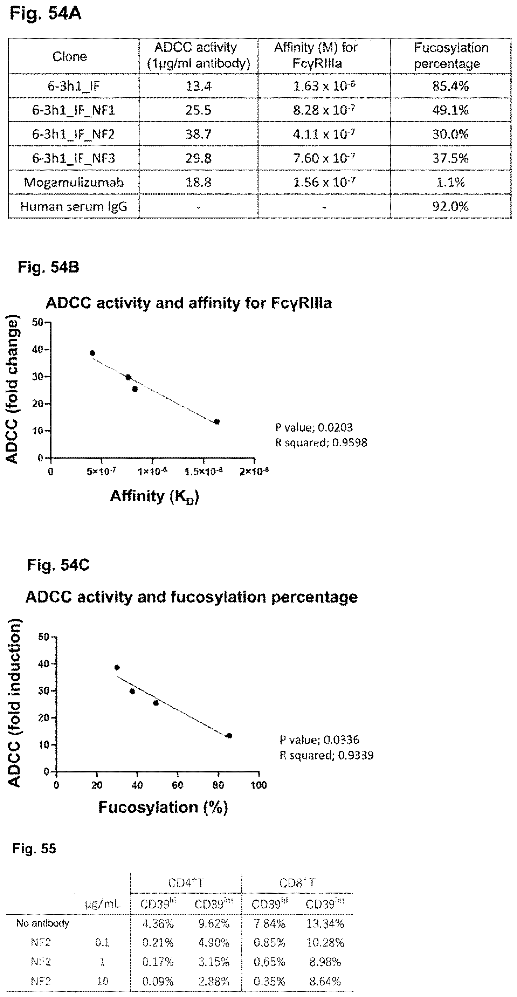

Figure 54A-C show tables and graphs summarizing the results of 16.1-16.2, 18.1-18.2, and 20.1-20.2 above. Strong correlations were observed between the ADCC activity value and the affinity for FcγRIIIa or the fucosylation percentage (R² = 0.9598 or 0.9339, respectively).

### 21.1 Effects on T cells

Purchased healthy human PBMCs (33000-10, Wako Pure Chemicals) were seeded at 5 × 10⁵ cells/well in a 96 well U-bottom plate and cultured in the presence of 100 IU/mL IL-2 (87-890, NIPRO) and 20 ng/mL IL-7 (200-07, PeproTech) for 5 or 6 days to induce Tregs. The medium was washed out and the cells were cultured in the presence of CMV peptide A02 (TS-0010-1c, MBL), 10 IU/mL IL-2, and 20 ng/mL IL-7 for 11 days to induce antigen-specific cytotoxic T cells. The medium used was RPMI-1640 (189-0202, Wako Pure Chemicals) supplemented with 10 % Human Serum AB (100-512, H46X00K, Gemini Bio), 1 mM penicillin streptomycin (09367-34, Nacalai Tesque), 1 mM sodium pyruvate (11360070 , Gibco), 10 mM HEPES (17557-9, Nacalai Tesque), 1% GlutaMAX (35050061, Thermo Fisher), and 0.01 mM 2-mercaptoethanol (21438-82, Nacalai Tesque).

FACSVerse (BD) was used for various cell type analyses. Cells were counted using CountBright^{™} Absolute Counting Beads (C36950, Invitrogen). GraphPad Prism 9.2.0 was used for data analysis. One-way ANOVA and Dunnet method were used for statistical analysis. p*≤0.05, p**≤0.01, p***≤0.001, or p****≤0.0001 was considered significantly different.

### 21.2 Results

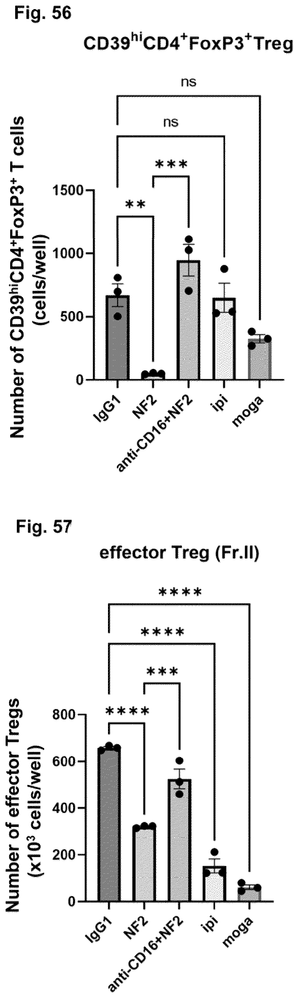

Figs. 55-59 show the results of cell type analysis, Treg removal evaluation, and cytotoxic T cell test. By culturing healthy PBMCs in the presence of 6-3h1_IF_NF2 (10 µg/mL) for 6 days, the CD39^{hi} fraction in CD4⁺ T cells or CD8⁺ T cells was selectively removed (Fig. 55). In addition, when healthy PBMCs were cultured in the presence of 6-3h1_IF_NF2 (10 µg/mL) for 5 days, CD39^{hi}CD4⁺FoxP3⁺ Tregs and effector Tregs (Fr. II, CD4⁺CD45RA-FoxP3^{hi}) disappeared significantly and markedly (Figs. 56- 57). Further, this disappearance was completely reversed by anti-CD16 (FcyRIIIa) antibody treatment. This indicates that the Treg removal effect of 6-3h1_IF_NF2 is based on CD16 (FcyRIIIa)-mediated ADCC.

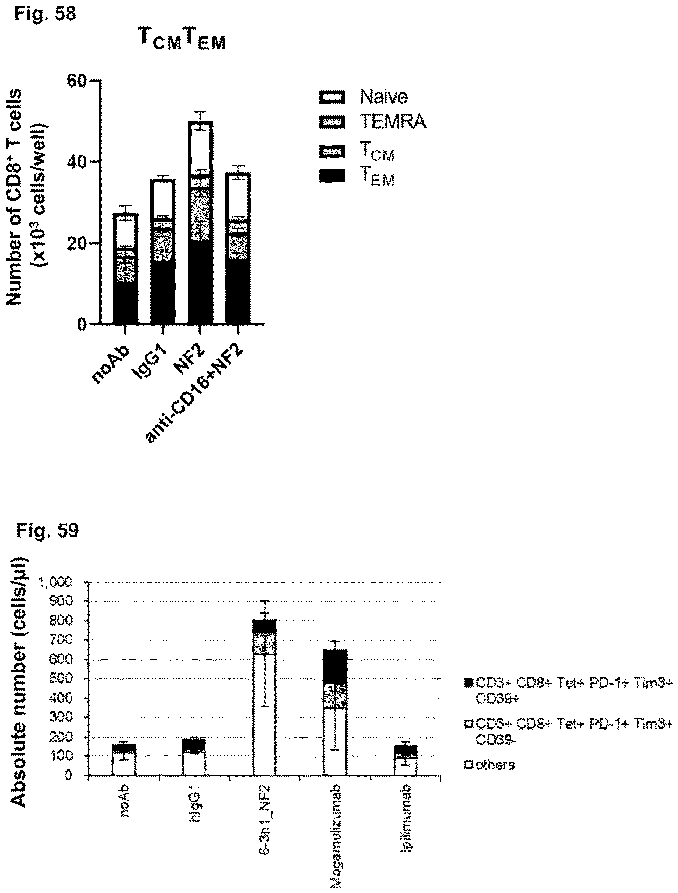

Furthermore, the number of CD8⁺ T_{CM} (central memory, CD8⁺CD45RA⁻CCR7⁻) and CD8⁺ T_{EM} (effector memory, CD8⁺CD45RA⁻CCR7⁺) cells were increased, which were completely abolished by anti-CD16 (FcyRIIIa) antibody treatment (Fig. 58).

Ipilimumab (ipi, YERVOY (registered trademark), Bristol Myers Squibb) and mogamulizumab (moga, POTELIGEO (registered trademark), Kyowa Kirin) induced a significant loss of only effector Tregs (Fr. II) (Figs. 56-57).

Here, 6-3h1_IF_NF2 (10 µg/mL) markedly induced antigen-specific cytotoxic T cells, with a lower percentage of CD39⁺/⁻PD1⁺Tim-3⁺ fraction than mogamulizumab, and their exhaustion was lower (Fig. 59).

### 22.1 Cancer cell growth inhibition test with Treg-removed PBMCs.

Purchased healthy human PBMCs (Wako Pure Chemicals, 33000-10M) were seeded at 5 × 10⁵ cells/well in a 96 well U-bottom plate and cultured in the presence of 100 IU/mL IL-2 (NIPRO, 87-890), 20 ng/mL IL-7 (PeproTech, 200-07), and each test antibody at 10 µg/ml for 5 days. Then, 15,000 cells/well of A375 (malignant melanoma cancer cell line) overexpressing CMV antigen and RFP were mixed with 45,000 cells/well of PBMCs prepared after 5 days of culture, and the mixture was co-cultured at 37°C and 5% CO₂ for 5 days. At this time, the medium used was RPMI-1640 (189-0202, Wako Pure Chemicals) supplemented with 10 % Human Serum AB (100-512, H46X00K, Gemini Bio), 1 mM penicillin streptomycin (09367-34, Nacalai Tesque), 1 mM sodium pyruvate (11360070 , Gibco), 10 mM HEPES (17557-9, Nacalai Tesque), 1% GlutaMAX (35050061, Thermo Fisher), and 0.01 mM 2-mercaptoethanol (21438-82, Nacalai Tesque). The number of A375 cells was then determined by measuring the fluorescence value of RFP while using a Varioskan LUX multimode microplate reader (Thermo Fisher Scientific). GraphPad Prism 9.2.0 was used for data analysis. One-way ANOVA and Dunnet method were used for statistical analysis. p****≤0.0001 was considered significantly different.

### 22.2 Results

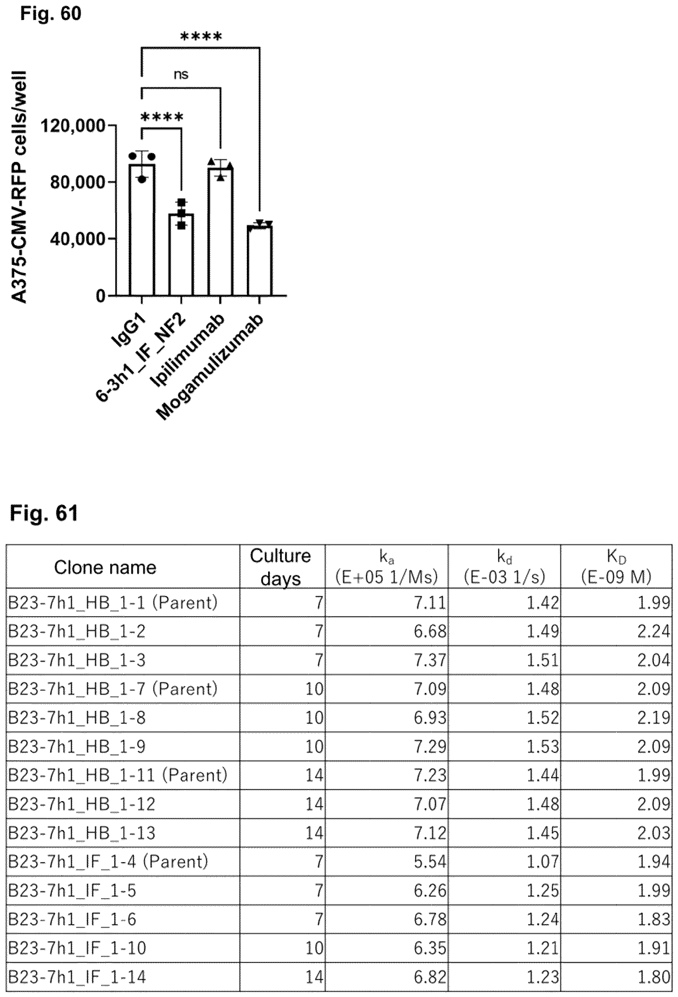

Fig. 60 shows the results. Here, 6-3h1_IF_NF2 (10 µg/mL)-treated PBMCs significantly inhibited the growth of human malignant melanoma A375. The anti-CTLA-4 antibody Ipilimumab exerted no inhibitory effect.

### 23.1 To prepare glycan-modified antibodies of B23-7h1_HB and B23-7h1_IF

First, an expression vector was created by recombining the heavy-chain constant region sequence of the expression vector containing B23-7h4_HB or B23-7h4_IF from human CH (IgG4SP) to human CH (IgG1), thereby changing the subclass from IgG4 to IgG1. The antibody expressed from this vector was named B23-7h1_HB or B23-7h1_IF (the VH and VL amino acid sequences are the same as those of B23-7h4_HB or B23-7h4_IF; the amino acid sequence of human CH (IgG1) is represented by SEQ ID NO: 88). Next, the following procedure was used to produce glycan-modified antibodies prepared under multiple conditions for each clone. The above expression vector was then transfected into CHO cells by using an ExpiCHO Expression System Kit (Thermo Fisher Scientific, A29133) to generate transiently antibody-expressing CHO cells. Here, 2F-Peracetyl-Fucose (DMSO suspension) (Merck, 344827) at a final concentration of 20 µM was added in culture the day before and/or 3-10 days after transfection to prepare each antibody. The cells were cultured according to the instructions of the ExpiCHO Expression System Kit, and the corresponding antibody produced in the culture supernatant from the CHO cells was collected and purified 7-14 days after transfection. The above procedure was used to obtain the glycan-modified B23-7h1_HB or glycan-modified B23-7h1_IF antibody listed in the clone name column of Fig. 61. The antibodies prepared without the addition of 2F-Peracetyl-Fucose were denoted as "Parent".

In addition, the glycan-modified 6-3h1_IF was prepared by the procedure for preparing 6-3h1_IF_NF2 described in the Section 13.1 above. The antibody collected on day 7 of culture after transfection was designated 6-3h1_IF_NF2_D7, and the antibody collected on day 13 was designated 6-3h1_IF_NF2_D13.

### 23.2 Affinity for recombinant CD39

Substantially the same procedure as described in the Section 2.1 above was used to measure the affinity of glycan-modified B23-7h1_HB or glycan-modified B23-7h1_IF antibody for human CD39.

### 23.3 Results

Fig. 61 shows the results of SPR analysis. The affinity for human CD39 was almost the same K_{D} value regardless of the presence or absence of glycan modification or the glycan modification method.

### 24.1 Affinity for FcyRIIIa (V variant)

Substantially the same procedure as described in the Section 20.1 above was used to prepare solutions of each test antibody by 5-step dilution starting from 250 × 10⁻⁹ M, and then measure the affinity of each antibody for FcyRIIIa (V variant).

### 24.2 Results

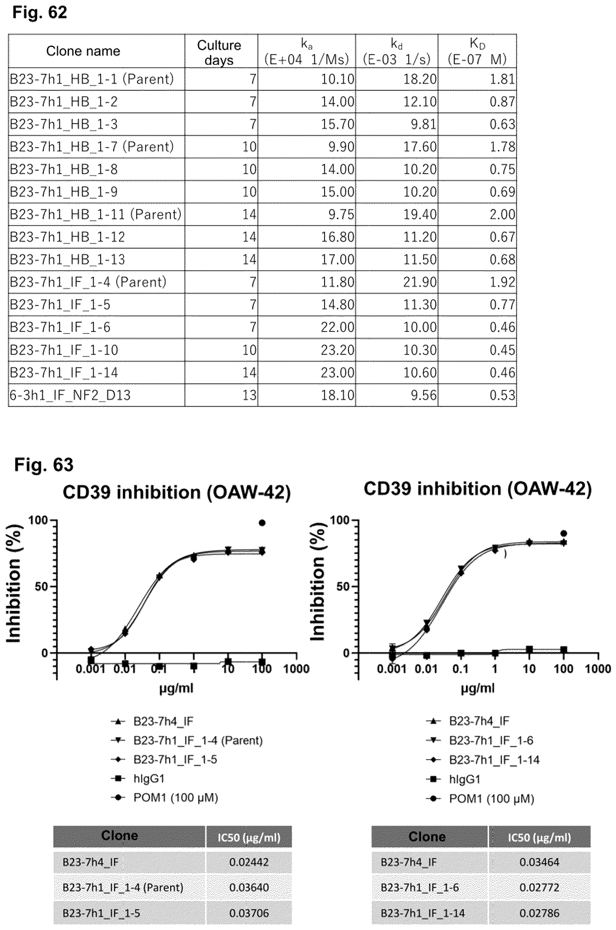

Fig. 62 shows the results of SPR analysis. The affinity of the parent antibody for FcyRIIIa was unchanged, but the affinity of each glycan-modified antibody for FcyRIIIa changed depending on the preparation method, and each antibody showed higher affinity than the parent antibody.

### 25.1 Activity to inhibit CD39 enzyme on human ovarian cancer cell line OAW-42

Substantially the same procedure as described in the Section 1.8 above was used to measure the activity of B23-7h4_IF, B23-7h1_IF, or each glycan-modified antibody thereof to inhibit human CD39.

### 25.2 Results

Fig. 63 shows the results of measuring the enzyme inhibitory activity. The human CD39 inhibitory activity was almost the same value regardless of the antibody subclass, the presence or absence of glycan modification, or the glycan modification method. The two B23-7h4_IF results were the results obtained using different plates.

### 26.1 Glycan analysis

Substantially the same method as described in the Section 16.1 above was used to measure the fucosylation percentage by glycan analysis.

### 26.2 Results

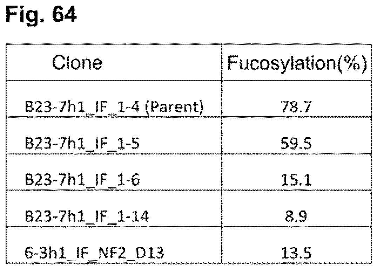

Fig. 64 shows the results of glycan analysis. The fucosylation percentages of B23-7h1_IF_1-4 (Parent), B23-7h1_IF_1-5, B23-7h1_IF _1-6, B23-7h1_IF_1-14 and 6-3h1_IF_NF2_D13 were 78.7%, 59.5%, 15.1%, 8.9% and 13.5%, respectively. Core fucose addition in each glycan-modified antibody was suppressed, with B23-7h1_IF_1-14 being the most suppressed.

### 27.1 ADCC reporter assay (Tregs as target and OAW-42 as target)

Substantially the same method as described in the Section 18.1 above was used to measure the activity while changing the concentration of each test antibody added after seeding 1.5 × 10⁴ or 3 × 10⁴ cells/well of activated Tregs or the human ovarian cancer line OAW-42 as target cells.

### 27.2 Results

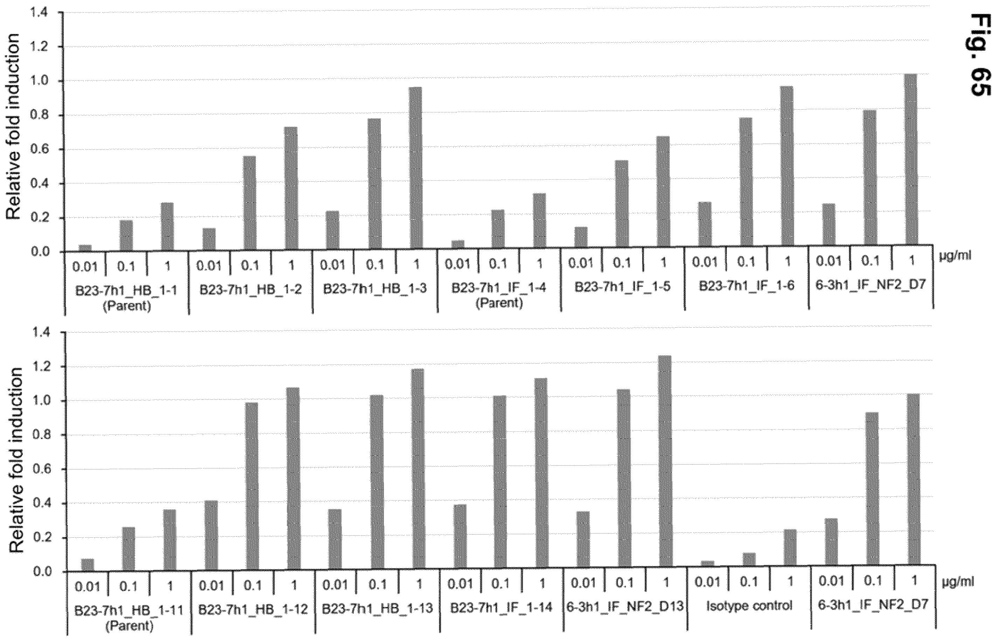

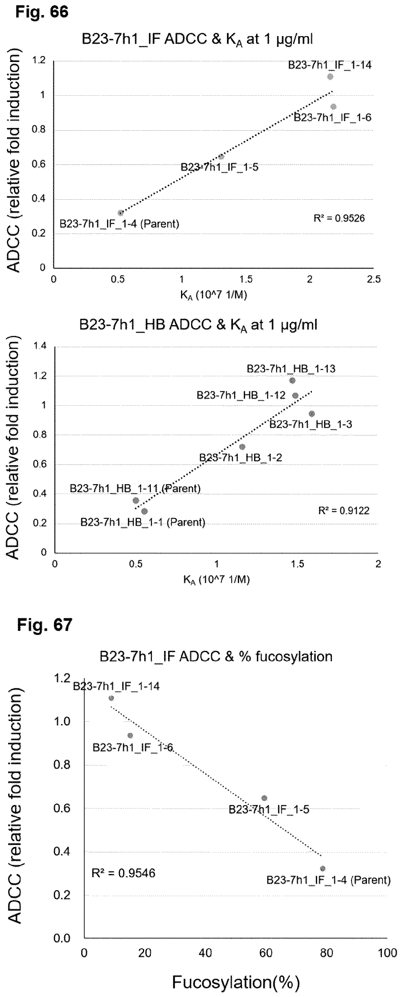

Fig. 65 shows the results of the ADCC activity assay (using Tregs as a target). In the assay using activated Tregs as a target, each glycan-modified antibody showed varied ADCC activity depending on the preparation method. The two 6-3h1_IF_NF2_D7 results were the results obtained using different plates. Fig. 66 shows the correlation between the ADCC activity and the FcyRIIIa binding activity, and Fig. 67 shows the correlation between the ADCC activity and the fucosylation percentage. A strong correlation was observed between the ADCC activity and the affinity for FcyRIIIa (K_{A} value is the reciprocal of K_{D} value), and between the ADCC activity and the fucosylation percentage.

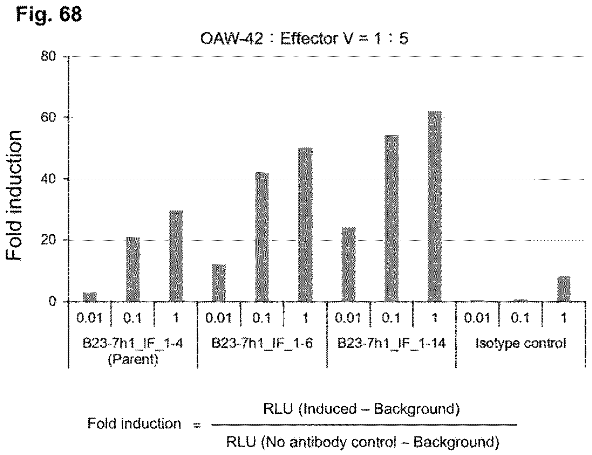

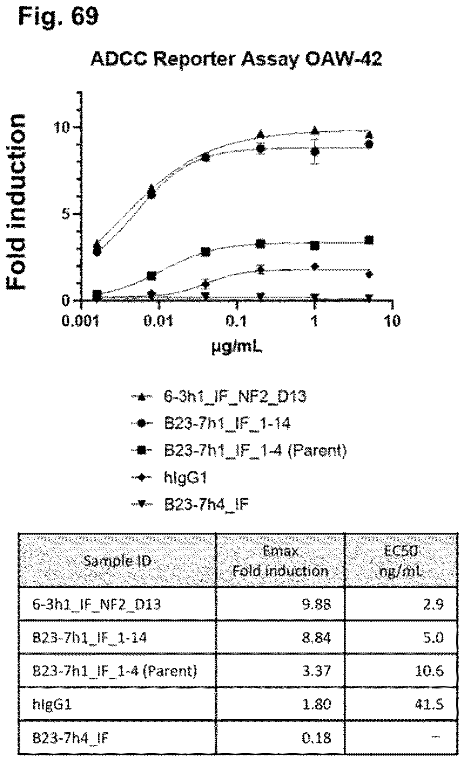

Figs. 68 and 69 show the results of the ADCC activity assay (using OAW-42 as a target). The results have demonstrated that the maximum activity of B23-7h1_IF_1-14 in the assay using OAW-42 as the target cells corresponded to the ADCC induction rate about 2.6 times higher that that of B23-7h1_IF_1-4 (Parent).

### 28.1 To selectively remove CD39^{hi} T cells

Substantially the same method as described in the Section 21.1 above was used to check a change in the percentage of various CD39^{hi}-expressing T cells by each glycan-modified anti-CD39 antibody.

### 28.2 Results

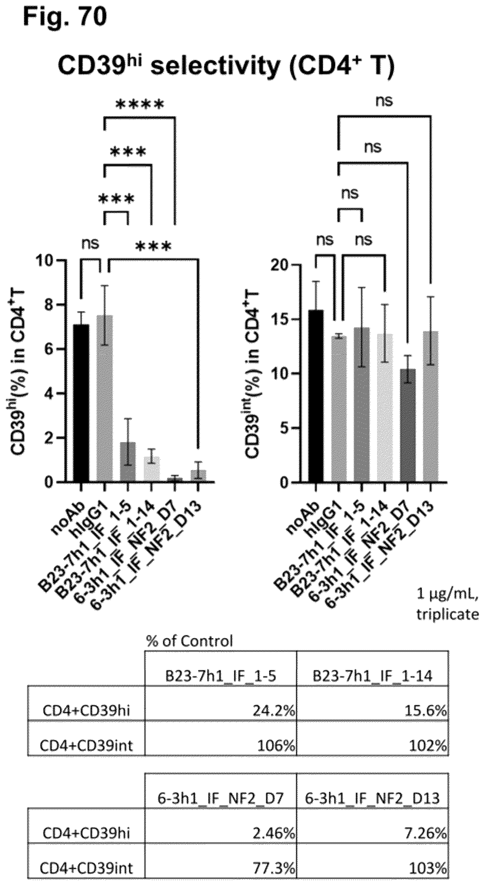

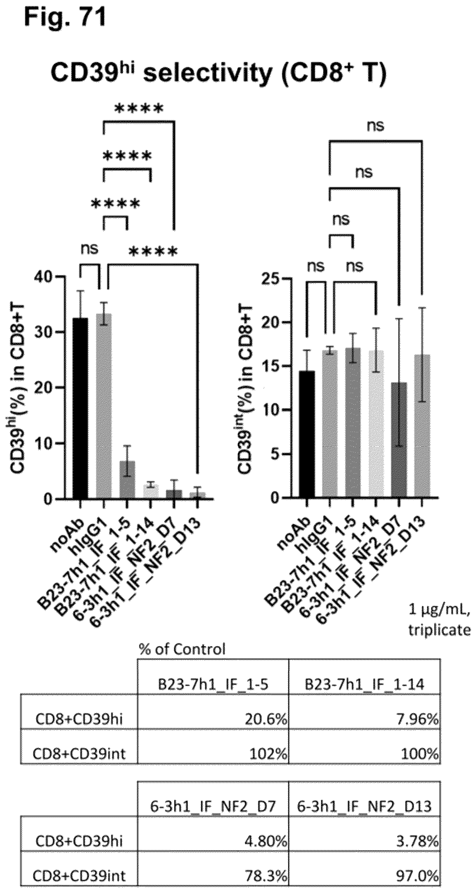

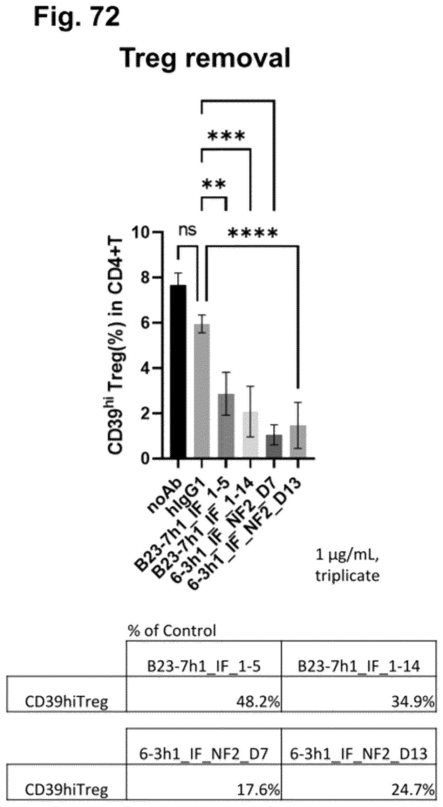

Figs. 70-72 show the results of the cell type analysis. The CD39^{hi} fraction in CD4⁺ or CD8⁺ T cells was significantly reduced, and the CD39^{int} fraction exhibited no significant change. This has indicated the ability of each clone to selectively remove CD39^{hi} cells.

As described in the above Examples, the present inventors have obtained novel anti-CD39 antibodies. Furthermore, the obtained anti-CD39 antibodies were shown to have remarkable properties for use in cancer therapy.

Hereinabove, the present invention has been described based on the Examples. The Examples are just examples. Those skilled in the art should understand that various modifications are allowed and such modified embodiments are within the scope of the present invention.

**Sequence Listing**

## Claims

1. An antibody or antigen-binding fragment that binds to CD39, comprising:
(a) heavy chain CDR1 comprising an amino acid sequence set forth in SEQ ID NO: 1, heavy chain CDR2 comprising an amino acid sequence set forth in SEQ ID NO: 2, and heavy chain CDR3 comprising an amino acid sequence set forth in SEQ ID NO: 3 and light chain CDR1 comprising an amino acid sequence set forth in SEQ ID NO: 4, light chain CDR2 comprising an amino acid sequence set forth in SEQ ID NO: 5, and light chain CDR3 comprising an amino acid sequence set forth in SEQ ID NO: 6;
(b) heavy chain CDR1 comprising an amino acid sequence set forth in SEQ ID NO: 7, heavy chain CDR2 comprising an amino acid sequence set forth in SEQ ID NO: 8, and heavy chain CDR3 comprising an amino acid sequence set forth in SEQ ID NO: 9 and light chain CDR1 comprising an amino acid sequence set forth in SEQ ID NO: 10, light chain CDR2 comprising an amino acid sequence set forth in SEQ ID NO: 11, and light chain CDR3 comprising an amino acid sequence set forth in SEQ ID NO: 12;
(c) heavy chain CDR1 comprising an amino acid sequence set forth in SEQ ID NO: 13, heavy chain CDR2 comprising an amino acid sequence set forth in SEQ ID NO: 14, and heavy chain CDR3 comprising an amino acid sequence set forth in SEQ ID NO: 15 and light chain CDR1 comprising an amino acid sequence set forth in SEQ ID NO: 16, light chain CDR2 comprising an amino acid sequence set forth in SEQ ID NO: 17, and light chain CDR3 comprising an amino acid sequence set forth in SEQ ID NO: 18;
(d) heavy chain CDR1 comprising an amino acid sequence set forth in SEQ ID NO: 19, heavy chain CDR2 comprising an amino acid sequence set forth in SEQ ID NO: 20, and heavy chain CDR3 comprising an amino acid sequence set forth in SEQ ID NO: 21and light chain CDR1 comprising an amino acid sequence set forth in SEQ ID NO: 22, light chain CDR2 comprising an amino acid sequence set forth in SEQ ID NO: 23 and light chain CDR3 comprising an amino acid sequence set forth in SEQ ID NO: 24;
(e) heavy chain CDR1 comprising an amino acid sequence set forth in SEQ ID NO: 25, heavy chain CDR2 comprising an amino acid sequence set forth in SEQ ID NO: 26, and heavy chain CDR3 comprising an amino acid sequence set forth in SEQ ID NO: 27 and light chain CDR1 comprising an amino acid sequence set forth in SEQ ID NO: 28, light chain CDR2 comprising an amino acid sequence set forth in SEQ ID NO: 29 and light chain CDR3 comprising an amino acid sequence set forth in SEQ ID NO: 30;
(f) heavy chain CDR1 comprising an amino acid sequence set forth in SEQ ID NO: 31, heavy chain CDR2 comprising an amino acid sequence set forth in SEQ ID NO: 32, and heavy chain CDR3 comprising an amino acid sequence set forth in SEQ ID NO: 33 and light chain CDR1 comprising an amino acid sequence set forth in SEQ ID NO: 34, light chain CDR2 comprising an amino acid sequence set forth in SEQ ID NO: 35 and light chain CDR3 comprising an amino acid sequence set forth in SEQ ID NO: 36; or
(g) heavy chain CDR1 comprising an amino acid sequence set forth in SEQ ID NO: 37, heavy chain CDR2 comprising an amino acid sequence set forth in SEQ ID NO: 38, and heavy chain CDR3 comprising an amino acid sequence set forth in SEQ ID NO: 39 and light chain CDR1 comprising an amino acid sequence set forth in SEQ ID NO: 40, light chain CDR2 comprising an amino acid sequence set forth in SEQ ID NO: 41 and light chain CDR3 comprising an amino acid sequence set forth in SEQ ID NO: 42.

2. The antibody or antigen-binding fragment according to claim 1, which inhibits CD39 activity.

3. The antibody or antigen-binding fragment according to claim 1 or 2, which inhibits growth of malignant tumor cells.

4. The antibody or antigen-binding fragment according to any one of claims 1 to 3, which promotes cytotoxicity of immune cells.

5. The antibody or antigen-binding fragment according to any one of claims 1 to 4, which is a monoclonal antibody.

6. The antibody or antigen-binding fragment according to any one of claims 1 to 5, wherein the antibody is a mouse, chimeric, or humanized antibody.

7. The antibody or antigen-binding fragment according to any one of claims 1 to 6, wherein K_{D} (M) toward CD39 is 9.0 × 10⁻⁹ or less.

8. The antibody or antigen-binding fragment according to any one of claims 1 to 7, which is a glycan-modified antibody.

9. A polynucleotide or vector that encodes the antibody or antigen-binding fragment according to any one of claims 1 to 8.

10. A cell comprising the polynucleotide or vector according to claim 9.

11. A method for producing an antibody or antigen-binding fragment, comprising the step of growing the cell according to claim 10.

12. A composition comprising the antibody or antigen-binding fragment according to any one of claims 1 to 8.

13. A pharmaceutical composition for treatment of malignant tumor, comprising the antibody or antigen-binding fragment according to any one of claims 1 to 8.

14. A composition for inhibiting CD39 activity, comprising the antibody or antigen-binding fragment according to any one of claims 1 to 8.

15. A composition for promoting TNFα production by immune cells, comprising the antibody or antigen-binding fragment according to any one of claims 1 to 8.

16. A composition for promoting cytotoxicity, comprising the antibody or antigen-binding fragment according to any one of claims 1 to 8.

17. A pharmaceutical composition comprising the antibody or antigen-binding fragment according to any one of claims 1 to 8, wherein the antibody or antigen-binding fragment has an N-type glycan, and 40% or more of the N-type glycan in the pharmaceutical composition is free of core fucose.

18. A pharmaceutical composition for treatment of malignant tumor, comprising the antibody or antigen-binding fragment according to any one of claims 1 to 8, wherein the pharmaceutical composition is used in combination therapy with a chemotherapeutic agent and the antibody or antigen-binding fragment according to any one of claims 1 to 8.

19. A pharmaceutical composition for treatment of malignant tumor, comprising a chemotherapeutic agent, wherein the pharmaceutical composition is used in combination therapy with the chemotherapeutic agent and the antibody or antigen-binding fragment according to any one of claims 1 to 8.

20. A kit comprising the antibody or antigen-binding fragment according to any one of claims 1 to 8.

21. A composition for selectively removing CD39^{hi} cells, comprising an anti-CD39 antibody.
